(19) Europäisches Patentamt
European Patent Office
Office européen des brevets

(11) Publication number: **0 619 301 A2**

# (12) EUROPEAN PATENT APPLICATION

(21) Application number: **94105186.4**

(22) Date of filing: **31.03.94**

(51) Int. Cl.[5]: **C07C 271/06**, C07C 271/28, C07C 333/12, C07C 327/58, A01N 47/24

(30) Priority: **04.04.93 JP 101919/93**

(43) Date of publication of application: **12.10.94 Bulletin 94/41**

(84) Designated Contracting States: **CH DE ES FR GB IT LI**

(71) Applicant: **NIHON NOHYAKU CO., LTD.**
**1-2-5, Nihonbashi**
**Chuo-ku Tokyo (JP)**

(72) Inventor: **Ohnishi, Masanobu**
**2-27-201, Kamihotsumi-1-chome**
**Ibaragi-shi, Osaka-fu (JP)**
Inventor: **Tajima, Sohkichi**
**10-9-301, Nagai-2-chome,**
**Sumiyosho-ku**
**Osaka-shi, Osaka-fu (JP)**
Inventor: **Yamamoto, Yoshinobu**
**40-1-625, Mikanodai-1-chome**
**Kawachinagano-shi (JP)**
Inventor: **Kanno, Hideo**
**2-2-708, Shirakawa-3-chome**
**Ibaraki-shi (JP)**

(74) Representative: **Patentanwälte Grünecker, Kinkeldey, Stockmair & Partner**
**Maximilianstrasse 58**
**D-80538 München (DE)**

## (54) N-Substituted phennylcarbamic acid derivates a process for production thereof, and agricultural and horticultural fungicides.

(57) An N-substituted phenylcarbamic acid derivative represented by the general formula:

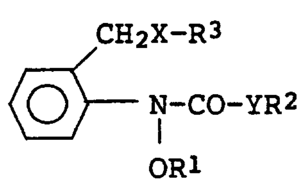

(I)

[wherein as disclosed in the specification, each of $R^1$ and $R^2$ is an alkyl group or the like, X is O, S or $N(R^8)$, and $R^3$ is $-N=C(R^4)R^5$ or the like in the case of X being O], a process for producing said derivative, and an agricultural and horticultural fungicide containing said derivative as an active ingredient.

EP 0 619 301 A2

BACKGROUND OF THE INVENTION

Field of the Invention

The present invention relates to N-substituted phenylcarbamic acid derivatives represented by the following general formula (I), a process for producing the same, and agricultural and horticultural fungicides:

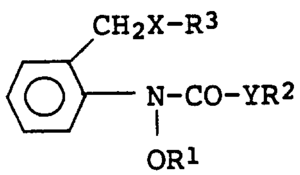

(I)

wherein $R^1$ is a hydrogen atom, a $C_{1-6}$alkyl group, a halo-$C_{1-6}$alkyl group, a $C_{1-6}$alkoxy-$C_{1-6}$alkyl group, a $C_{2-6}$alkenyl group, a $C_{2-6}$alkynyl group or a halo-$C_{1-6}$alkoxy-$C_{1-6}$alkyl group, $R^2$ is a hydrogen atom, a $C_{1-6}$alkyl group, a halo-$C_{1-6}$alkyl group, a $C_{1-6}$alkoxy-$C_{1-6}$alkyl group, a $C_{2-6}$alkenyl group, a $C_{2-6}$alkynyl group, a halo-$C_{1-6}$alkoxy-$C_{1-6}$alkyl group or a cyano-$C_{1-6}$alkyl group, X is an oxygen atom, a sulfur atom or a group represented by the formula:

$N(R^8)$

(wherein $R^8$ is a formyl group, a $C_{1-6}$alkyl group, a halo-$C_{1-6}$alkyl group, a $C_{1-6}$alkylcarbonyl group, a halo-$C_{1-6}$alkylcarbonyl group, a $C_{1-6}$alkoxycarbonyl group, a $C_{1-6}$alkoxy-$C_{1-6}$alkylcarbonyl group or a $C_{1-6}$alkylsulfonyl group), $R^3$ is as follows:

in the case of X being an oxygen atom, $R^3$ is a group represented by the formula:

$-N=C(R^4)R^5$

(wherein $R^4$ and $R^5$ are as follows:

in the case of $R^4$ being a $C_{1-6}$alkylthio group, $R^5$ is a hydrogen atom; a cyano group; a $C_{1-6}$alkyl group; a halo-$C_{1-6}$alkyl group; a $C_{3-6}$cycloalkyl group; a halo-$C_{3-6}$cycloalkyl group; a $C_{3-6}$cycloalkyl-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; a $C_{1-6}$alkoxy-$C_{1-6}$alkyl group; a $C_{1-6}$alkylthio-$C_{1-6}$alkyl group; a $C_{2-6}$alkenyl group; a halo-$C_{2-6}$alkenyl group; a $C_{3-6}$cycloalkenyl group; a $C_{2-6}$alkynyl group; a $C_{1-6}$alkylcarbonyl group; a $C_{1-6}$alkoxycarbonyl group; an unsubstituted phenyl group; a substituted phenyl group having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylcarbonyl groups, $C_{1-6}$alkoxycarbonyl groups, di-$C_{1-6}$alkylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; an unsubstituted phenoxy group; a substituted phenoxy group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio group; a substituted phenylthio group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkyl group; a substituted phenyl-$C_{1-6}$alkyl group having on the ring 1 to 5 substituents which may be the same or different and are selected from the group

consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{2-6}$alkenyl group; a substituted phenyl-$C_{2-6}$alkenyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylcarbonyl group; a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxycarbonyl group; a substituted phenoxycarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxy-$C_{1-6}$alkyl group; a substituted phenoxy-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio-$C_{1-6}$alkyl group; a substituted phenylthio-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylthio group; a substituted phenyl-$C_{1-6}$alkylthio group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylcarbonyl group; a substituted phenyl-$C_{1-6}$alkylcarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkoxycarbonyl group; a substituted phenyl-$C_{1-6}$alkoxycarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group, and

in the case of $R^4$ being a $C_{1-6}$alkyl group, $R^5$ is an unsubstituted phenylcarbonyl group or a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene groups),

in the case of X being a sulfur atom, $R^3$ is a group represented by the formula:

$$-C(X^1-R^6)=N-R^7$$

(wherein $X^1$ is an oxygen atom or a sulfur atom, $R^6$ is a $C_{1-6}$alkyl group, a halo-$C_{1-6}$alkyl group, a $C_{2-6}$alkenyl group, a $C_{2-6}$alkynyl group or a $C_{1-6}$alkoxy-$C_{1-6}$alkyl group, and $R^7$ is an unsubstituted

phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, and substituted phenyl groups having 1 to 5 substituents which may be the same or different and are selected from the group consisting of halogen atoms and $C_{1-6}$alkyl groups),

in the case of X being a group represented by the formula:

$N(R^8)$

(wherein $R^8$ is as defined above), $R^3$ is a group represented by the formula:

$-N=C(R^9)R^{10}$

(wherein $R^9$ and $R^{10}$, which may be the same or different, are hydrogen atoms; cyano groups; $C_{1-6}$alkyl groups; halo-$C_{1-6}$alkyl groups; $C_{3-6}$cycloalkyl groups; halo-$C_{3-6}$cycloalkyl groups; $C_{3-6}$cycloalkyl-$C_{1-6}$alkyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkoxy-$C_{1-6}$alkyl groups; $C_{1-6}$alkylthio-$C_{1-6}$alkyl groups; $C_{2-6}$alkenyl groups; halo-$C_{2-6}$alkenyl groups; $C_{2-6}$cycloalkenyl groups; $C_{2-6}$alkynyl groups; halo-$C_{2-6}$alkynyl groups; $C_{1-6}$alkylcarbonyl groups; $C_{1-6}$alkoxycarbonyl groups; unsubstituted phenyl groups; substituted phenyl groups having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{2-6}$alkenyloxy groups, halo-$C_{2-6}$alkenyloxy groups, $C_{2-6}$alkynyloxy groups, halo-$C_{2-6}$alkynyloxy groups, $C_{1-6}$alkylcarbonyl groups, $C_{1-6}$alkoxycarbonyl groups, di-$C_{1-6}$alkylamino groups, di-$C_{2-6}$alkenylamino groups, di-$C_{2-6}$alkynylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; unsubstituted phenoxy groups; substituted phenoxy groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio groups; substituted phenylthio groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkyl groups; substituted phenyl-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{2-6}$alkenyl groups; substituted phenyl-$C_{2-6}$alkenyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylcarbonyl groups; substituted phenylcarbonyl groups having 1 to 5 substituents which may be the same or different and are selected from the group consisting of

halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxycarbonyl groups; substituted phenoxycarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxy-$C_{1-6}$alkyl groups; substituted phenoxy-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio-$C_{1-6}$alkyl groups; substituted phenylthio-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylthio groups; substituted phenyl-$C_{1-6}$alkylthio groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylcarbonyl groups; substituted phenyl-$C_{1-6}$alkylcarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkoxycarbonyl groups; substituted phenyl-$C_{1-6}$alkoxycarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$-alkylenedioxy groups; 5- to 7-membered heterocyclic rings having 1 to 3 heteroatoms which may be the same or different and are selected from the group consisting of oxygen atom, sulfur atom and nitrogen atom; heterocyclic rings having a benzene ring condensed therewith; or heterocyclic rings having a $C_{3-6}$cyclo-alkane group condensed therewith; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group), and Y is an oxygen atom or a sulfur atom.

## Related Art

EP Laid-Open No. 0498396 and WO93/15046 disclose that N-phenylcarbamate derivatives are useful as agricultural and horticultural fungicides.

## DETAILED DESCRIPTION OF THE PREFERRED EMBODIMENTS

In the definition of the substituents of the N-substituted phenylcarbamic acid derivative of the general formula (I) of the present invention, the prefix "halo" is used for expressing that a group contains one or more halogen atoms selected from chlorine, fluorine, bromine and iodine atoms. For example, the term "haloalkyl group" means a substituted alkyl group having as the substituent(s) one or more halogen atoms which may be the same or different and are selected from the group consisting of chlorine atom, fluorine atom, bromine atom and iodine atom.

Preferable examples of substituent for $R^1$ are alkyl groups such as methyl, ethyl, n-propyl, i-propyl, n-butyl, s-butyl, i-butyl, t-butyl, n-pentyl, n-hexyl, etc. Of these, methyl group and ethyl group are particularly preferable.

Preferable examples of substituent for $R^2$ are alkyl groups such as methyl, ethyl, n-propyl, i-propyl, n-butyl, s-butyl, i-butyl, t-butyl, n-pentyl, n-hexyl, etc. Of these, methyl group is particulary preferable.

Preferable examples of substituent for $R^3$ are as follows.

When X is O, $R^3$ is $-N=C(R^4)R^5$. Preferable examples of substituent for $R^4$ are alkyl groups such as methyl, ethyl, n-propyl, i-propyl, n-butyl, s-butyl, i-butyl, t-butyl, n-pentyl, n-hexyl, etc., and alkylthio groups such as methylthio, ethylthio, n-propylthio, i-propylthio, n-butylthio, s-butylthio, i-butylthio, t-butylthio, n-pentylthio, n-hexylthio, etc. Of these, three alkyl groups, i.e., methyl, ethyl and propyl groups, and two alkylthio groups, i.e., methylthio and ethylthio groups are particularly preferable.

Preferable examples of substituent for $R^5$ are an unsubstituted phenyl group; substituted phenyl groups having 1 to 5 substituents which may be the same or different and are selected from the group consisting of halogen atoms, $C_{1-6}$alkyl groups and $C_{1-6}$alkoxy groups; heterocyclic rings having 1 to 3 heteroatoms which may be the same or different and are selected from the group consisting of oxygen atom, sulfur atom and nitrogen atom; heterocyclic rings having a benzene ring condensed therewith; cycloalkane rings; such heterocyclic rings or cycloalkane rings, which have 1 to 5 substituents which may be the same or different and are selected from the group consisting of halogen atoms, $C_{1-6}$alkyl groups and $C_{1-6}$alkoxy groups; an unsubstituted phenylcarbonyl group; and substituted phenylcarbonyl groups having 1 to 5 substituents which may be the same or different and are selected from the group consisting of halogen atoms, $C_{1-6}$alkyl groups and $C_{1-6}$alkoxy groups.

When X is S, $R^3$ is $-C(X^1-R^6)=N-R^7$. A preferable example of substituent for $X^1$ is a sulfur atom. Preferable examples of substituent for $R^6$ are alkyl groups such as methyl, ethyl, n-propyl, i-propyl, n-butyl, s-butyl, i-butyl, t-butyl, n-pentyl, n-hexyl, etc. Preferable examples of substituent for $R^7$ are an unsubstituted phenyl group and substituted phenyl groups having 1 to 5 substituents selected from the group consisting of $C_{1-6}$alkyl groups, halo-$C_{1-6}$alkyl groups and $C_{1-6}$alkoxy groups.

When X is $-N(R^8)-$ (wherein $R^8$ is preferably a $C_{1-6}$alkylcarbonyl group), $R^3$ is $-N=C(R^9)R^{10}$. Preferable examples of substituents for $R^9$ and $R^{10}$, respectively, which may be the same or different are alkyl groups such as methyl, ethyl, n-propyl, i-propyl, n-butyl, s-butyl, i-butyl, t-butyl, n-pentyl, n-hexyl, etc.; unsubstituted phenyl group, substituted phenyl groups having 1 to 5 substituents selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups and $C_{1-6}$alkylthio groups; 5- or 6-membered heterocyclic rings having heteroatoms which may be the same or different and are selected from the group consisting of oxygen atom, sulfur atom and nitrogen atom; and said 5- or 6-membered heterocyclic rings which have 1 to 4 substituents selected from halogen atoms.

The N-substituted phenylcarbamic acid derivative of the general formula (I) of the present invention can be produced, for example, by the following process.

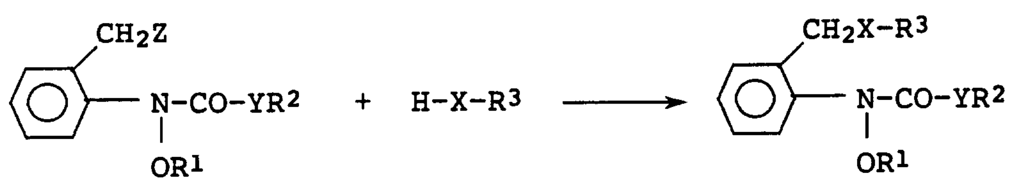

(III) (II) (I)

wherein $R^1$, $R^2$, $R^3$, X, and Y are as defined above and Z is a halogen atom.

The N-substituted phenylcarbamic acid derivative of the general formula (I) can be produced by reacting a compound of the general formula (III) with a compound of the general formula (II) in the presence of a base or a silver compound, and an inert solvent.

As the inert solvent usable in this reaction, any inert solvent may be used so long as it does not markedly inhibit the progress of the reaction. There may be used, for example, alcohols such as isopropanol, tert-butanol, diethylene glycol, etc.; ketones such as acetone, methyl ethyl ketone, cyclohexanone, etc.; ethers such as diethyl ether, diisopropyl ether, tetrahydrofuran, dioxane, monoglyme, diglyme, etc.; halogenated hydrocarbons such as dichloroethane, chloroform, carbon tetrachloride, tetrachloroethane, etc.; aromatic hydrocarbons such as benzene, chlorobenzene, nitrobenzene, toluene, etc.; nitriles such as acetonitrile, etc.; dimethylformamide; dimethyl sulfoxide; and water. These inert solvents may be used singly or as a mixture thereof.

Two-phase reaction can be carried out as the above reaction by using water and a water-insoluble inert solvent. In this case, there can be used phase transfer catalysts such as triethylbenzylammonium chloride, trioctylmethylammonium chloride, etc.

As the base used in the reaction, an inorganic base or an organic base may be used. As the inorganic base, there may be used, for example, carbonates or hydroxides of alkali metal atoms or alkaline earth metal atoms, such as sodium carbonate, potassium carbonate, calcium carbonate, sodium hydrogencarbonate, sodium hydroxide, potassium hydroxide, calcium hydroxide, etc., and hydrides of alkali metal atoms, such as lithium hydride, sodium hydride, etc. As the organic base, there may be used, for example, alkoxides of alkali metal atoms, such as sodium methoxide, potassium tert-butoxide, etc., diethylamine, triethylamine, pyridine, and benzyltrimethylammonium hydroxide. These bases may be used singly or as a mixture thereof. The amount of the base used may be properly chosen in the range of 1 mole to excess moles per mole of the compound of the general formula (III).

As the silver compound usable in the reaction, silver oxide, for example, may be used. The amount of the silver compound used may be properly chosen in the range of 1 mole to excess moles per mole of the compound of the general formula (III).

Since the reaction is an equimolar reaction, it is sufficient that the compound of the general formula (III) and the compound of the general formula (II) are used in equimolar amounts, though either of them may be used in excess.

The reaction temperature is properly chosen in the range of -70 ° C to the boiling point of the inert solvent used. It is preferably -40 ° C to room temperature.

Although the reaction time is varied depending on the reaction temperature, the degree of reaction, etc., it is usually chosen in the range of 30 minutes to 48 hours.

After completion of the reaction, the desired compound is isolated from the reaction mixture by a conventional method, and if necessary, purified by column chromatography, recrystallization, etc., whereby the N-substituted phenylcarbamic acid derivative of the general formula (I) can be produced.

Typical compounds as the N-substituted phenylcarbamic acid derivative of the general formula (I) are divided into three groups, compounds of of general formula (I)-1, compounds of general formula (I)-2 and compounds of general formula (I)-3 and are listed below, but they are not intended in any way to limit the scope of the present invention.

General formula (1)-1

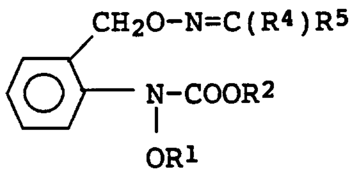

(I)-1

Table 1 (wherein Ph is a phenyl group)

| No. | $R^1$ | $R^2$ | $R^4$ | $R^5$ | Physical property |
|---|---|---|---|---|---|
| 1 | $CH_3$ | $CH_3$ | $SCH_3$ | Ph | |
| 2 | $CH_3$ | $CH_3$ | $SCH_3$ | 2-F-Ph | nD 1.5634 (20.5°C) |
| 3 | $CH_3$ | $CH_3$ | $SCH_3$ | 3-F-Ph | |
| 4 | $CH_3$ | $CH_3$ | $SCH_3$ | 4-F-Ph | nD 1.5712 (20.6°C) |
| 5 | $CH_3$ | $CH_3$ | $SCH_3$ | 2-Cl-Ph | |
| 6 | $CH_3$ | $CH_3$ | $SCH_3$ | 3-Cl-Ph | |
| 7 | $CH_3$ | $CH_3$ | $SCH_3$ | 4-Cl-Ph | nD 1.5795 (20.8°C) |
| 8 | $CH_3$ | $CH_3$ | $SCH_3$ | 3-Br-Ph | nD 1.5888 (20.4°C) |
| 9 | $CH_3$ | $CH_3$ | $SCH_3$ | 2-Br-Ph | |
| 10 | $CH_3$ | $CH_3$ | $SCH_3$ | 4-Br-Ph | nD 1.6011 (19.3°C) |
| 11 | $CH_3$ | $CH_3$ | $SCH_3$ | 2-$CH_3$-Ph | nD 1.5593 (22.2°C) |
| 12 | $CH_3$ | $CH_3$ | $SCH_3$ | 3-$CH_3$-Ph | |
| 13 | $CH_3$ | $CH_3$ | $SCH_3$ | 4-$CH_3$-Ph | nD 1.5759 (19.0°C) |
| 14 | $CH_3$ | $CH_3$ | $SCH_3$ | 2-$CF_3$-Ph | |

- Cont'd -

Table 1 (Cont'd)

| No. | $R^1$ | $R^2$ | $R^4$ | $R^5$ | Physical property |
|---|---|---|---|---|---|
| 15 | $CH_3$ | $CH_3$ | $SCH_3$ | $3-CF_3-Ph$ | nD 1.5513 (16.6°C) |
| 16 | $CH_3$ | $CH_3$ | $SCH_3$ | $4-CF_3-Ph$ | nD 1.5486 (19.6°C) |
| 17 | $CH_3$ | $CH_3$ | $SCH_3$ | $2-CH_3O-Ph$ | |
| 18 | $CH_3$ | $CH_3$ | $SCH_3$ | $3-CH_3O-Ph$ | |
| 19 | $CH_3$ | $CH_3$ | $SCH_3$ | $4-CH_3O-Ph$ | |
| 20 | $CH_3$ | $CH_3$ | $SCH_3$ | $2-CF_3O-Ph$ | |
| 21 | $CH_3$ | $CH_3$ | $SCH_3$ | $3-CF_3O-Ph$ | |
| 22 | $CH_3$ | $CH_3$ | $SCH_3$ | $4-CF_3O-Ph$ | |
| 23 | $CH_3$ | $CH_3$ | $SCH_3$ | $3,4-Cl_2-Ph$ | nD 1.5980 (16.6°C) |
| 24 | $CH_3$ | $CH_3$ | $SCH_3$ | $3,5-Cl_2-Ph$ | nD 1.5490 (18.3°C) |
| 25 | $CH_3$ | $CH_3$ | $SCH_3$ | $2,6-Cl_2-Ph$ | nD 1.5326 (17.9°C) |
| 26 | $CH_3$ | $CH_3$ | $SCH_3$ | $2,4-(CH_3O)_2-Ph$ | |
| 27 | $CH_3$ | $CH_3$ | $SCH_3$ | $3,4-(CH_3O)_2-Ph$ | nD 1.5121 (17.3°C) |
| 28 | $CH_3$ | $CH_3$ | $SCH_3$ | $3,5-(CH_3O)_2-Ph$ | |
| 29 | $CH_3$ | $CH_3$ | $SCH_3$ | $2,4,5-(Cl)_3-Ph$ | |
| 30 | $CH_3$ | $CH_3$ | $SCH_3$ | $2,4,6-(Cl)_3-Ph$ | |
| 31 | $CH_3$ | $CH_3$ | $SCH_3$ | $2,4,5-(CH_3)_3-Ph$ | nD 1.5597 (21.1°C) |
| 32 | $CH_3$ | $CH_3$ | $SCH_3$ | $2,4,6-(CH_3)_3-Ph$ | nD 1.5692 (18.2°C) |
| 33 | $CH_3$ | $CH_3$ | $SC_2H_5$ | Ph | |
| 34 | $CH_3$ | $CH_3$ | $SC_2H_5$ | 2-F-Ph | |
| 35 | $CH_3$ | $CH_3$ | $SC_2H_5$ | 3-F-Ph | |
| 36 | $CH_3$ | $CH_3$ | $SC_2H_5$ | 4-F-Ph | |

- Cont'd -

Table 1 (Cont'd)

| No. | $R^1$ | $R^2$ | $R^4$ | $R^5$ | Physical property |
|---|---|---|---|---|---|
| 37 | $CH_3$ | $CH_3$ | $SC_2H_5$ | 2-Cl-Ph | |
| 38 | $CH_3$ | $CH_3$ | $SC_2H_5$ | 3-Cl-Ph | |
| 39 | $CH_3$ | $CH_3$ | $SC_2H_5$ | 4-Cl-Ph | |
| 40 | $CH_3$ | $CH_3$ | $SC_2H_5$ | 2-Br-Ph | |
| 41 | $CH_3$ | $CH_3$ | $SC_2H_5$ | 3-Br-Ph | |
| 42 | $CH_3$ | $CH_3$ | $SC_2H_5$ | 4-Br-Ph | |
| 43 | $CH_3$ | $CH_3$ | $SC_2H_5$ | 2-$CH_3$-Ph | |
| 44 | $CH_3$ | $CH_3$ | $SC_2H_5$ | 3-$CH_3$-Ph | |
| 45 | $CH_3$ | $CH_3$ | $SC_2H_5$ | 4-$CH_3$-Ph | |
| 46 | $CH_3$ | $CH_3$ | $SC_2H_5$ | 2-$CF_3$-Ph | |
| 47 | $CH_3$ | $CH_3$ | $SC_2H_5$ | 3-$CF_3$-Ph | |
| 48 | $CH_3$ | $CH_3$ | $SC_2H_5$ | 4-$CF_3$-Ph | |
| 49 | $CH_3$ | $CH_3$ | $SC_2H_5$ | 2-$CH_3O$-Ph | |
| 50 | $CH_3$ | $CH_3$ | $SC_2H_5$ | 3-$CH_3O$-Ph | |
| 51 | $CH_3$ | $CH_3$ | $SC_2H_5$ | 4-$CH_3O$-Ph | |
| 52 | $CH_3$ | $CH_3$ | $SC_2H_5$ | 2-$CF_3O$-Ph | |
| 53 | $CH_3$ | $CH_3$ | $SC_2H_5$ | 3-$CF_3O$-Ph | |
| 54 | $CH_3$ | $CH_3$ | $SC_2H_5$ | 4-$CF_3O$-Ph | |
| 55 | $CH_3$ | $CH_3$ | $SC_2H_5$ | 3,4-$Cl_2$-Ph | |
| 56 | $CH_3$ | $CH_3$ | $SC_2H_5$ | 3,5-$Cl_2$-Ph | |
| 57 | $CH_3$ | $CH_3$ | $SC_2H_5$ | 2,6-$Cl_2$-Ph | |
| 58 | $CH_3$ | $CH_3$ | $SC_2H_5$ | 2,4-$(CH_3O)_2$-Ph | |

- Cont'd -

Table 1 (Cont'd)

| No. | $R^1$ | $R^2$ | $R^4$ | $R^5$ | Physical property |
|---|---|---|---|---|---|
| 59 | $CH_3$ | $CH_3$ | $SC_2H_5$ | 3,4-$(CH_3O)_2$-Ph | |
| 60 | $CH_3$ | $CH_3$ | $SC_2H_5$ | 3,5-$(CH_3O)_2$-Ph | |
| 61 | $CH_3$ | $CH_3$ | $SC_2H_5$ | 2,4,5-$(Cl)_3$-Ph | |
| 62 | $CH_3$ | $CH_3$ | $SC_2H_5$ | 2,4,6-$(Cl)_3$-Ph | |
| 63 | $CH_3$ | $CH_3$ | $SC_2H_5$ | 2,4,5-$(CH_3)_3$-Ph | |
| 64 | $CH_3$ | $CH_3$ | $SC_2H_5$ | 2,4,6-$(CH_3)_3$-Ph | |
| 65 | $CH_3$ | $CH_3$ | $SC_2H_5$ | Indane-5-yl | |
| 66 | $C_2H_5$ | $CH_3$ | $SCH_3$ | Ph | |
| 67 | $C_2H_5$ | $CH_3$ | $SCH_3$ | 2-F-Ph | |
| 68 | $C_2H_5$ | $CH_3$ | $SCH_3$ | 3-F-Ph | |
| 69 | $C_2H_5$ | $CH_3$ | $SCH_3$ | 4-F-Ph | |
| 70 | $C_2H_5$ | $CH_3$ | $SCH_3$ | 2-Cl-Ph | |
| 71 | $C_2H_5$ | $CH_3$ | $SCH_3$ | 3-Cl-Ph | |
| 72 | $C_2H_5$ | $CH_3$ | $SCH_3$ | 4-Cl-Ph | |
| 73 | $C_2H_5$ | $CH_3$ | $SCH_3$ | 2-Br-Ph | |
| 74 | $C_2H_5$ | $CH_3$ | $SCH_3$ | 3-Br-Ph | |
| 75 | $C_2H_5$ | $CH_3$ | $SCH_3$ | 4-Br-Ph | |
| 76 | $C_2H_5$ | $CH_3$ | $SCH_3$ | 2-$CH_3$-Ph | |
| 77 | $C_2H_5$ | $CH_3$ | $SCH_3$ | 3-$CH_3$-Ph | |
| 78 | $C_2H_5$ | $CH_3$ | $SCH_3$ | 4-$CH_3$-Ph | |
| 79 | $C_2H_5$ | $CH_3$ | $SCH_3$ | 2-$CF_3$-Ph | |
| 80 | $C_2H_5$ | $CH_3$ | $SCH_3$ | 3-$CF_3$-Ph | |

- Cont'd -

Table 1 (Cont'd)

| No. | $R^1$ | $R^2$ | $R^4$ | $R^5$ | Physical property |
|---|---|---|---|---|---|
| 81 | $C_2H_5$ | $CH_3$ | $SCH_3$ | 4-$CF_3$-Ph | |
| 82 | $C_2H_5$ | $CH_3$ | $SCH_3$ | 2-$CH_3O$-Ph | |
| 83 | $C_2H_5$ | $CH_3$ | $SCH_3$ | 3-$CH_3O$-Ph | |
| 84 | $C_2H_5$ | $CH_3$ | $SCH_3$ | 4-$CH_3O$-Ph | |
| 85 | $C_2H_5$ | $CH_3$ | $SCH_3$ | 2-$CF_3O$-Ph | |
| 86 | $C_2H_5$ | $CH_3$ | $SCH_3$ | 3-$CF_3O$-Ph | |
| 87 | $C_2H_5$ | $CH_3$ | $SCH_3$ | 4-$CF_3O$-Ph | |
| 88 | $C_2H_5$ | $CH_3$ | $SCH_3$ | 3,4-$Cl_2$-Ph | |
| 89 | $C_2H_5$ | $CH_3$ | $SCH_3$ | 3,5-$Cl_2$-Ph | |
| 90 | $C_2H_5$ | $CH_3$ | $SCH_3$ | 2,6-$Cl_2$-Ph | |
| 91 | $C_2H_5$ | $CH_3$ | $SCH_3$ | 2,4-$(CH_3O)_2$-Ph | |
| 92 | $C_2H_5$ | $CH_3$ | $SCH_3$ | 3,4-$(CH_3O)_2$-Ph | |
| 93 | $C_2H_5$ | $CH_3$ | $SCH_3$ | 3,5-$(CH_3O)_2$-Ph | |
| 94 | $C_2H_5$ | $CH_3$ | $SCH_3$ | 2,4,5-$(Cl)_3$-Ph | |
| 95 | $C_2H_5$ | $CH_3$ | $SCH_3$ | 2,4,6-$(Cl)_3$-Ph | |
| 96 | $C_2H_5$ | $CH_3$ | $SCH_3$ | 2,4,5-$(Cl)_3$-Ph | |
| 97 | $C_2H_5$ | $CH_3$ | $SCH_3$ | 2,4,6-$(CH_3)_3$-Ph | |
| 98 | $C_2H_5$ | $CH_3$ | $SCH_3$ | Indane-5-yl | |
| 99 | $CH_3$ | $C_2H_5$ | $SC_2H_5$ | Ph | |
| 100 | $CH_3$ | $C_2H_5$ | $SC_2H_5$ | 2-F-Ph | |
| 101 | $CH_3$ | $C_2H_5$ | $SC_2H_5$ | 3-F-Ph | |
| 102 | $CH_3$ | $C_2H_5$ | $SC_2H_5$ | 4-F-Ph | |

- Cont'd -

Table 1 (Cont'd)

| No. | $R^1$ | $R^2$ | $R^4$ | $R^5$ | Physical property |
|---|---|---|---|---|---|
| 103 | $CH_3$ | $C_2H_5$ | $SC_2H_5$ | 2-Cl-Ph | |
| 104 | $CH_3$ | $C_2H_5$ | $SC_2H_5$ | 3-Cl-Ph | |
| 105 | $CH_3$ | $C_2H_5$ | $SC_2H_5$ | 4-Cl-Ph | |
| 106 | $CH_3$ | $C_2H_5$ | $SC_2H_5$ | 2-Br-Ph | |
| 107 | $CH_3$ | $C_2H_5$ | $SC_2H_5$ | 3-Br-Ph | |
| 108 | $CH_3$ | $C_2H_5$ | $SC_2H_5$ | 4-Br-Ph | |
| 109 | $CH_3$ | $C_2H_5$ | $SC_2H_5$ | 2-$CH_3$-Ph | |
| 110 | $CH_3$ | $C_2H_5$ | $SC_2H_5$ | 3-$CH_3$-Ph | |
| 111 | $CH_3$ | $C_2H_5$ | $SC_2H_5$ | 4-$CH_3$-Ph | |
| 112 | $CH_3$ | $C_2H_5$ | $SC_2H_5$ | 2-$CF_3$-Ph | |
| 113 | $CH_3$ | $C_2H_5$ | $SC_2H_5$ | 3-$CF_3$-Ph | |
| 114 | $CH_3$ | $C_2H_5$ | $SC_2H_5$ | 4-$CF_3$-Ph | |
| 115 | $CH_3$ | $C_2H_5$ | $SC_2H_5$ | 2-$CH_3O$-Ph | |
| 116 | $CH_3$ | $C_2H_5$ | $SC_2H_5$ | 3-$CH_3O$-Ph | |
| 117 | $CH_3$ | $C_2H_5$ | $SC_2H_5$ | 4-$CH_3O$-Ph | |
| 118 | $CH_3$ | $C_2H_5$ | $SC_2H_5$ | 2-$CF_3O$-Ph | |
| 119 | $CH_3$ | $C_2H_5$ | $SC_2H_5$ | 3-$CF_3O$-Ph | |
| 120 | $CH_3$ | $C_2H_5$ | $SC_2H_5$ | 4-$CF_3O$-Ph | |
| 121 | $CH_3$ | $C_2H_5$ | $SC_2H_5$ | 3,4-$Cl_2$-Ph | |
| 122 | $CH_3$ | $C_2H_5$ | $SC_2H_5$ | 3,5-$Cl_2$-Ph | |
| 123 | $CH_3$ | $C_2H_5$ | $SC_2H_5$ | 2,6-$Cl_2$-Ph | |
| 124 | $CH_3$ | $C_2H_5$ | $SC_2H_5$ | 2,4-$(CH_3O)_2$-Ph | |

- Cont'd -

Table 1 (Cont'd)

| No. | $R^1$ | $R^2$ | $R^4$ | $R^5$ | Physical property |
|---|---|---|---|---|---|
| 125 | $CH_3$ | $C_2H_5$ | $SC_2H_5$ | 3,4-$(CH_3O)_2$-Ph | |
| 126 | $CH_3$ | $C_2H_5$ | $SC_2H_5$ | 3,5-$(CH_3O)_2$-Ph | |
| 127 | $CH_3$ | $C_2H_5$ | $SC_2H_5$ | 2,4,5-$(Cl)_3$-Ph | |
| 128 | $CH_3$ | $C_2H_5$ | $SC_2H_5$ | 2,4,6-$(Cl)_3$-Ph | |
| 129 | $CH_3$ | $C_2H_5$ | $SC_2H_5$ | 2,4,5-$(CH_3)_3$-Ph | |
| 130 | $CH_3$ | $C_2H_5$ | $SC_2H_5$ | 2,4,6-$(CH_3)_3$-Ph | |
| 131 | $CH_3$ | $C_2H_5$ | $SC_2H_5$ | Indane-5-yl | |
| 132 | $CH_3$ | $CH_3$ | $CH_3$ | Ph-CO | |
| 133 | $CH_3$ | $CH_3$ | $CH_3$ | 2-Cl-Ph-CO | |
| 134 | $CH_3$ | $CH_3$ | $CH_3$ | 3-Cl-Ph-CO | |
| 135 | $CH_3$ | $CH_3$ | $CH_3$ | 4-Cl-Ph-CO | |
| 136 | $CH_3$ | $CH_3$ | $CH_3$ | 2-F-Ph-CO | |
| 137 | $CH_3$ | $CH_3$ | $CH_3$ | 3-F-Ph-CO | |
| 138 | $CH_3$ | $CH_3$ | $CH_3$ | 4-F-Ph-CO | |
| 139 | $CH_3$ | $CH_3$ | $CH_3$ | 2-Br-Ph-CO | |
| 140 | $CH_3$ | $CH_3$ | $CH_3$ | 3-Br-Ph-CO | |
| 141 | $CH_3$ | $CH_3$ | $CH_3$ | 4-Br-Ph-CO | |
| 142 | $CH_3$ | $CH_3$ | $CH_3$ | 2,4-$Cl_2$-Ph-CO | |
| 143 | $CH_3$ | $CH_3$ | $CH_3$ | 3,4-$Cl_2$-Ph-CO | |
| 144 | $CH_3$ | $CH_3$ | $CH_3$ | 2,4-$F_2$-Ph-CO | |
| 145 | $CH_3$ | $CH_3$ | $CH_3$ | 3,4-$F_2$-Ph-CO | |
| 146 | $CH_3$ | $CH_3$ | $CH_3$ | 2-$CH_3$-Ph-CO | |

- Cont'd -

Table 1 (Cont'd)

| No. | $R^1$ | $R^2$ | $R^4$ | $R^5$ | Physical property |
|---|---|---|---|---|---|
| 147 | $CH_3$ | $CH_3$ | $CH_3$ | 3-$CH_3$-Ph-CO | |
| 148 | $CH_3$ | $CH_3$ | $CH_3$ | 4-$CH_3$-Ph-CO | |
| 149 | $CH_3$ | $CH_3$ | $CH_3$ | 2-$CF_3$-Ph-CO | |
| 150 | $CH_3$ | $CH_3$ | $CH_3$ | 3-$CF_3$-Ph-CO | |
| 151 | $CH_3$ | $CH_3$ | $CH_3$ | 4-$CF_3$-Ph-CO | |
| 152 | $CH_3$ | $CH_3$ | $CH_3$ | 2-$CH_3O$-Ph-CO | |
| 153 | $CH_3$ | $CH_3$ | $CH_3$ | 3-$CH_3O$-Ph-CO | |
| 154 | $CH_3$ | $CH_3$ | $CH_3$ | 3,4-$(CH_3O)_2$-Ph-CO | nD 1.5634 (20.9°C) |
| 155 | $CH_3$ | $CH_3$ | $CH_3$ | 2-$CF_3O$-Ph-CO | |
| 156 | $CH_3$ | $CH_3$ | $CH_3$ | 3-$CF_3O$-Ph-CO | |
| 157 | $CH_3$ | $CH_3$ | $CH_3$ | 4-$CF_3O$-Ph-CO | |
| 158 | $CH_3$ | $CH_3$ | $CH_3$ | 2-$CH_3S$-Ph-CO | |
| 159 | $CH_3$ | $CH_3$ | $CH_3$ | 3-$CH_3S$-Ph-CO | |
| 160 | $CH_3$ | $CH_3$ | $CH_3$ | 4-$CH_3S$-Ph-CO | |
| 161 | $CH_3$ | $CH_3$ | $CH_3$ | 2-$CF_3S$-Ph-CO | |
| 162 | $CH_3$ | $CH_3$ | $CH_3$ | 2-$CF_3S$-Ph-CO | |
| 163 | $CH_3$ | $CH_3$ | $CH_3$ | 3-$CF_3S$-Ph-CO | |
| 164 | $CH_3$ | $CH_3$ | $CH_3$ | 4-$CF_3S$-Ph-CO | |
| 165 | $CH_3$ | $CH_3$ | $CH_3$ | Indane-5-yl-CO | nD 1.5738 (20.9°C) |

General formula (1)-2

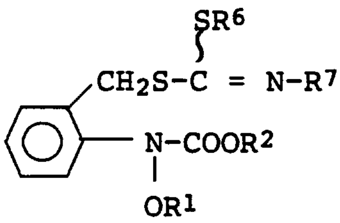

(I)-2

Table 2 (wherein Ph is a phenyl group)

| No. | $R^1$ | $R^2$ | $R^6$ | $R^7$ | Physical property |
|---|---|---|---|---|---|
| 166 | $CH_3$ | $CH_3$ | $CH_3$ | Ph | |
| 167 | $CH_3$ | $CH_3$ | $CH_3$ | 2-Cl-Ph | |
| 168 | $CH_3$ | $CH_3$ | $CH_3$ | 3-Cl-Ph | |
| 169 | $CH_3$ | $CH_3$ | $CH_3$ | 4-Cl-Ph | nD 1.6096 (28.3°C) |
| 170 | $CH_3$ | $CH_3$ | $CH_3$ | 2-F-Ph | |
| 171 | $CH_3$ | $CH_3$ | $CH_3$ | 3-F-Ph | |
| 172 | $CH_3$ | $CH_3$ | $CH_3$ | 4-F-Ph | |
| 173 | $CH_3$ | $CH_3$ | $CH_3$ | 2-Br-Ph | |
| 174 | $CH_3$ | $CH_3$ | $CH_3$ | 3-Br-Ph | |
| 175 | $CH_3$ | $CH_3$ | $CH_3$ | 4-Br-Ph | nD 1.6230 (23.8°C) |
| 176 | $CH_3$ | $CH_3$ | $CH_3$ | 2-$CH_3$-Ph | |
| 177 | $CH_3$ | $CH_3$ | $CH_3$ | 3-$CH_3$-Ph | |
| 178 | $CH_3$ | $CH_3$ | $CH_3$ | 4-$CH_3$-Ph | nD 1.6038 (24.0°C) |

- Cont'd -

Table 2 (Cont'd)

| No. | $R^1$ | $R^2$ | $R^6$ | $R^7$ | Physical property |
|---|---|---|---|---|---|
| 179 | $CH_3$ | $CH_3$ | $CH_3$ | 2-$CF_3$-Ph | |
| 180 | $CH_3$ | $CH_3$ | $CH_3$ | 3-$CF_3$-Ph | |
| 181 | $CH_3$ | $CH_3$ | $CH_3$ | 4-$CF_3$-Ph | |
| 182 | $CH_3$ | $CH_3$ | $CH_3$ | 2-$CH_3O$-Ph | |
| 183 | $CH_3$ | $CH_3$ | $CH_3$ | 3-$CH_3O$-Ph | |
| 184 | $CH_3$ | $CH_3$ | $CH_3$ | 4-$CH_3O$-Ph | nD 1.6129 (21.6°C) |
| 185 | $CH_3$ | $CH_3$ | $CH_3$ | 2-$CF_3O$-Ph | |
| 186 | $CH_3$ | $CH_3$ | $CH_3$ | 3-$CF_3O$-Ph | |
| 187 | $CH_3$ | $CH_3$ | $CH_3$ | 4-$CF_3O$-Ph | nD 1.5809 (20.7°C) |
| 188 | $CH_3$ | $CH_3$ | $CH_3$ | 3,5-$Cl_2$-Ph | paste |
| 189 | $CH_3$ | $CH_3$ | $CH_3$ | 3,4-$Cl_2$-Ph | |
| 190 | $CH_3$ | $CH_3$ | $CH_3$ | 2,4-$F_2$-Ph | nD 1.5613 (20.5°C) |
| 191 | $CH_3$ | $CH_3$ | $CH_3$ | 2,4-$(CH_3)_2$-Ph | nD 1.5890 (22.8°C) |
| 192 | $CH_3$ | $CH_3$ | $CH_3$ | 3,4-$(CH_3)_2$-Ph | nD 1.5138 (24.3°C) |
| 193 | $CH_3$ | $CH_3$ | $CH_3$ | 3,4-$(CH_3O)_2$-Ph | nD 1.5940 (17.8°C) |
| 194 | $CH_3$ | $CH_3$ | $CH_3$ | Pyridine-3-yl | nD 1.5159 (17.1°C) |
| 195 | $C_2H_5$ | $CH_3$ | $CH_3$ | Ph | |
| 196 | $C_2H_5$ | $CH_3$ | $CH_3$ | 2-Cl-Ph | |
| 197 | $C_2H_5$ | $CH_3$ | $CH_3$ | 3-Cl-Ph | |
| 198 | $C_2H_5$ | $CH_3$ | $CH_3$ | 4-Cl-Ph | |
| 199 | $C_2H_5$ | $CH_3$ | $CH_3$ | 2-F-Ph | |
| 200 | $C_2H_5$ | $CH_3$ | $CH_3$ | 3-F-Ph | |

- Cont'd -

Table 2 (Cont'd)

| No. | $R^1$ | $R^2$ | $R^6$ | $R^7$ | Physical property |
|---|---|---|---|---|---|
| 201 | $C_2H_5$ | $CH_3$ | $CH_3$ | 4-F-Ph | |
| 202 | $C_2H_5$ | $CH_3$ | $CH_3$ | 2-Br-Ph | |
| 203 | $C_2H_5$ | $CH_3$ | $CH_3$ | 3-Br-Ph | |
| 204 | $C_2H_5$ | $CH_3$ | $CH_3$ | 4-Br-Ph | |
| 205 | $C_2H_5$ | $CH_3$ | $CH_3$ | 2-$CH_3$-Ph | |
| 206 | $C_2H_5$ | $CH_3$ | $CH_3$ | 3-$CH_3$-Ph | |
| 207 | $C_2H_5$ | $CH_3$ | $CH_3$ | 4-$CH_3$-Ph | |
| 208 | $C_2H_5$ | $CH_3$ | $CH_3$ | 2-$CF_3$-Ph | |
| 209 | $C_2H_5$ | $CH_3$ | $CH_3$ | 3-$CF_3$-Ph | |
| 210 | $C_2H_5$ | $CH_3$ | $CH_3$ | 4-$CF_3$-Ph | |
| 211 | $C_2H_5$ | $CH_3$ | $CH_3$ | 2-$CH_3O$-Ph | |
| 212 | $C_2H_5$ | $CH_3$ | $CH_3$ | 3-$CH_3O$-Ph | |
| 213 | $C_2H_5$ | $CH_3$ | $CH_3$ | 4-$CH_3O$-Ph | nD 1.6080 (23.9°C) |
| 214 | $C_2H_5$ | $CH_3$ | $CH_3$ | 2-$CF_3O$-Ph | |
| 215 | $C_2H_5$ | $CH_3$ | $CH_3$ | 3-$CF_3O$-Ph | |
| 216 | $C_2H_5$ | $CH_3$ | $CH_3$ | 4-$CF_3O$-Ph | |
| 217 | $C_2H_5$ | $CH_3$ | $CH_3$ | 2,4-$Cl_2$-Ph | |
| 218 | $C_2H_5$ | $CH_3$ | $CH_3$ | 3,4-$Cl_2$-Ph | |
| 219 | $C_2H_5$ | $CH_3$ | $CH_3$ | 3,6-$Cl_2$-Ph | |
| 220 | $C_2H_5$ | $CH_3$ | $CH_3$ | 2,4-$(CH_3)_2$-Ph | |
| 221 | $C_2H_5$ | $CH_3$ | $CH_3$ | 3,4-$(CH_3)_2$-Ph | |
| 222 | $C_2H_5$ | $CH_3$ | $CH_3$ | 3,4-$(CH_3O)_2$-Ph | |

- Cont'd -

Table 2 (Cont'd)

| No. | $R^1$ | $R^2$ | $R^6$ | $R^7$ | Physical property |
|---|---|---|---|---|---|
| 223 | $C_2H_5$ | $CH_3$ | $CH_3$ | 2,4-$(CH_3O)_2$-Ph | |
| 224 | $CH_3$ | $C_2H_5$ | $CH_3$ | Ph | |
| 225 | $CH_3$ | $C_2H_5$ | $CH_3$ | 2-Cl-Ph | |
| 226 | $CH_3$ | $C_2H_5$ | $CH_3$ | 3-Cl-Ph | |
| 227 | $CH_3$ | $C_2H_5$ | $CH_3$ | 4-Cl-Ph | |
| 228 | $CH_3$ | $C_2H_5$ | $CH_3$ | 2-F-Ph | |
| 229 | $CH_3$ | $C_2H_5$ | $CH_3$ | 3-F-Ph | |
| 230 | $CH_3$ | $C_2H_5$ | $CH_3$ | 4-F-Ph | |
| 231 | $CH_3$ | $C_2H_5$ | $CH_3$ | 2-Br-Ph | |
| 232 | $CH_3$ | $C_2H_5$ | $CH_3$ | 3-Br-Ph | |
| 233 | $CH_3$ | $C_2H_5$ | $CH_3$ | 4-Br-Ph | |
| 234 | $CH_3$ | $C_2H_5$ | $CH_3$ | 2-$CH_3$-Ph | |
| 235 | $CH_3$ | $C_2H_5$ | $CH_3$ | 3-$CH_3$-Ph | |
| 236 | $CH_3$ | $C_2H_5$ | $CH_3$ | 4-$CH_3$-Ph | |
| 237 | $CH_3$ | $C_2H_5$ | $CH_3$ | 2-$CF_3$-Ph | |
| 328 | $CH_3$ | $C_2H_5$ | $CH_3$ | 3-$CF_3$-Ph | |
| 239 | $CH_3$ | $C_2H_5$ | $CH_3$ | 4-$CF_3$-Ph | |
| 240 | $CH_3$ | $C_2H_5$ | $CH_3$ | 2-$CH_3O$-Ph | |
| 241 | $CH_3$ | $C_2H_5$ | $CH_3$ | 3-$CH_3O$-Ph | |
| 242 | $CH_3$ | $C_2H_5$ | $CH_3$ | 4-$CH_3O$-Ph | |
| 243 | $CH_3$ | $C_2H_5$ | $CH_3$ | 2-$CF_3O$-Ph | |
| 244 | $CH_3$ | $C_2H_5$ | $CH_3$ | 3-$CF_3O$-Ph | |

- Cont'd -

Table 2 (Cont'd)

| No. | $R^1$ | $R^2$ | $R^6$ | $R^7$ | Physical property |
|---|---|---|---|---|---|
| 245 | $CH_3$ | $C_2H_5$ | $CH_3$ | 4-$CF_3O$-Ph | |
| 246 | $CH_3$ | $C_2H_5$ | $CH_3$ | 2,4-$Cl_2$-Ph | |
| 247 | $CH_3$ | $C_2H_5$ | $CH_3$ | 3,4-$Cl_2$-Ph | |
| 248 | $CH_3$ | $C_2H_5$ | $CH_3$ | 2,6-$Cl_2$-Ph | |
| 249 | $CH_3$ | $C_2H_5$ | $CH_3$ | 2,4-$(CH_3)_2$-Ph | |
| 250 | $CH_3$ | $C_2H_5$ | $CH_3$ | 3,4-$(CH_3)_2$-Ph | |
| 251 | $CH_3$ | $C_2H_5$ | $CH_3$ | 3,4-$(CH_3O)_2$-Ph | |
| 252 | $CH_3$ | $C_2H_5$ | $CH_3$ | 2,4-$(CH_3O)_2$-Ph | |
| 253 | $CH_3$ | $CH_3$ | $C_2H_5$ | Ph | |
| 254 | $CH_3$ | $CH_3$ | $C_2H_5$ | 2-Cl-Ph | |
| 255 | $CH_3$ | $CH_3$ | $C_2H_5$ | 3-Cl-Ph | |
| 256 | $CH_3$ | $CH_3$ | $C_2H_5$ | 4-Cl-Ph | |
| 257 | $CH_3$ | $CH_3$ | $C_2H_5$ | 2-F-Ph | |
| 258 | $CH_3$ | $CH_3$ | $C_2H_5$ | 3-F-Ph | |
| 259 | $CH_3$ | $CH_3$ | $C_2H_5$ | 4-F-Ph | |
| 260 | $CH_3$ | $CH_3$ | $C_2H_5$ | 2-Br-Ph | |
| 261 | $CH_3$ | $CH_3$ | $C_2H_5$ | 3-Br-Ph | |
| 262 | $CH_3$ | $CH_3$ | $C_2H_5$ | 4-Br-Ph | |
| 263 | $CH_3$ | $CH_3$ | $C_2H_5$ | 2-$CH_3$-Ph | |
| 264 | $CH_3$ | $CH_3$ | $C_2H_5$ | 3-$CH_3$-Ph | |
| 265 | $CH_3$ | $CH_3$ | $C_2H_5$ | 4-$CH_3$-Ph | |
| 266 | $CH_3$ | $CH_3$ | $C_2H_5$ | 2-$CF_3$-Ph | |

- Cont'd -

Table 2 (Cont'd)

| No. | $R^1$ | $R^2$ | $R^6$ | $R^7$ | Physical property |
|---|---|---|---|---|---|
| 267 | $CH_3$ | $CH_3$ | $C_2H_5$ | $3\text{-}CF_3\text{-}Ph$ | |
| 268 | $CH_3$ | $CH_3$ | $C_2H_5$ | $4\text{-}CF_3\text{-}Ph$ | |
| 269 | $CH_3$ | $CH_3$ | $C_2H_5$ | $2\text{-}CH_3O\text{-}Ph$ | |
| 270 | $CH_3$ | $CH_3$ | $C_2H_5$ | $3\text{-}CH_3O\text{-}Ph$ | |
| 271 | $CH_3$ | $CH_3$ | $C_2H_5$ | $4\text{-}CH_3O\text{-}Ph$ | |
| 272 | $CH_3$ | $CH_3$ | $C_2H_5$ | $2\text{-}CF_3O\text{-}Ph$ | |
| 273 | $CH_3$ | $CH_3$ | $C_2H_5$ | $3\text{-}CF_3O\text{-}Ph$ | |
| 274 | $CH_3$ | $CH_3$ | $C_2H_5$ | $4\text{-}CF_3O\text{-}Ph$ | |
| 275 | $CH_3$ | $CH_3$ | $C_2H_5$ | $2,4\text{-}Cl_2\text{-}Ph$ | |
| 276 | $CH_3$ | $CH_3$ | $C_2H_5$ | $3,4\text{-}Cl_2\text{-}Ph$ | |
| 277 | $CH_3$ | $CH_3$ | $C_2H_5$ | $2,6\text{-}Cl_2\text{-}Ph$ | |
| 278 | $CH_3$ | $CH_3$ | $C_2H_5$ | $2,4\text{-}(CH_3)_2\text{-}Ph$ | |
| 279 | $CH_3$ | $CH_3$ | $C_2H_5$ | $3,4\text{-}(CH_3)_2\text{-}Ph$ | |
| 280 | $CH_3$ | $CH_3$ | $C_2H_5$ | $3,4\text{-}(CH_3O)_2\text{-}Ph$ | |
| 281 | $CH_3$ | $CH_3$ | $C_2H_5$ | $2,4\text{-}(CH_3O)_2\text{-}Ph$ | |

General formula (1)-3

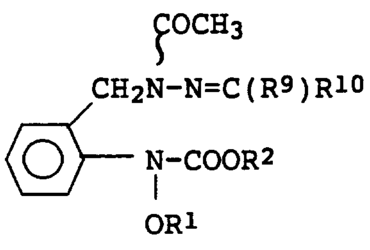

(I)-3

Table 3 (wherein Ph is a phenyl group)

| No. | $R^1$ | $R^2$ | $R^9$ | $R^{10}$ | Physical property |
|---|---|---|---|---|---|
| 282 | $CH_3$ | $CH_3$ | $CH_3$ | Ph | nD 1.5765 (19.5°C) |
| 283 | $CH_3$ | $CH_3$ | $CH_3$ | 2-F-Ph | |
| 284 | $CH_3$ | $CH_3$ | $CH_3$ | 3-F-Ph | nD 1.5654 (19.0°C) |
| 285 | $CH_3$ | $CH_3$ | $CH_3$ | 4-F-Ph | nD 1.5645 (22.6°C) |
| 286 | $CH_3$ | $CH_3$ | $CH_3$ | 2,4-$F_2$-Ph | nD 1.5534 (19.4°C) |
| 287 | $CH_3$ | $CH_3$ | $CH_3$ | 2,5-$F_2$-Ph | |
| 288 | $CH_3$ | $CH_3$ | $CH_3$ | 4-F-3-$NO_2$-Ph | nD 1.5682 (19.6°C) |
| 289 | $CH_3$ | $CH_3$ | $CH_3$ | 3,4-$F_2$-Ph | nD 1.5613 (18.7°C) |
| 290 | $CH_3$ | $CH_3$ | $CH_3$ | 2,6-$F_2$-Ph | nD 1.5479 (22.5°C) |
| 291 | $CH_3$ | $CH_3$ | $CH_3$ | 2,4,5-$F_3$-Ph | nD 1.5449 (23.9°C) |
| 292 | $CH_3$ | $CH_3$ | $CH_3$ | 2,4,6-$F_3$-Ph | |
| 293 | $CH_3$ | $CH_3$ | $CH_3$ | 2,3,4,5,6-$F_5$-Ph | |
| 294 | $CH_3$ | $CH_3$ | $CH_3$ | 2-Cl-Ph | |
| 295 | $CH_3$ | $CH_3$ | $CH_3$ | 3-Cl-Ph | nD 1.5814 (21.3°C) |
| 296 | $CH_3$ | $CH_3$ | $CH_3$ | 4-Cl-Ph | ND 1.5844 (15.6°C) |
| 297 | $CH_3$ | $CH_3$ | $CH_3$ | 2,3-$Cl_2$-Ph | |

- Cont'd -

Table 3 (Cont'd)

| No. | $R^1$ | $R^2$ | $R^9$ | $R^{10}$ | Physical property |
|---|---|---|---|---|---|
| 298 | $CH_3$ | $CH_3$ | $CH_3$ | 2,4-$Cl_2$-Ph | |
| 299 | $CH_3$ | $CH_3$ | $CH_3$ | 2,5-$Cl_2$-Ph | |
| 300 | $CH_3$ | $CH_3$ | $CH_3$ | 2,6-$Cl_2$-Ph | |
| 301 | $CH_3$ | $CH_3$ | $CH_3$ | 3,4-$Cl_2$-Ph | nD 1.5874 (21.7°C) |
| 302 | $CH_3$ | $CH_3$ | $CH_3$ | 3,5-$Cl_2$-Ph | |
| 303 | $CH_3$ | $CH_3$ | $CH_3$ | 2,4,5-$Cl_3$-Ph | |
| 304 | $CH_3$ | $CH_3$ | $CH_3$ | 3,4,5-$Cl_3$-Ph | |
| 305 | $CH_3$ | $CH_3$ | $CH_3$ | 2-Br-Ph | |
| 306 | $CH_3$ | $CH_3$ | $CH_3$ | 3-Br-Ph | nD 1.5752 (26.9°C) |
| 307 | $CH_3$ | $CH_3$ | $CH_3$ | 4-Br-Ph | nD 1.6025 (7.8°C) |
| 308 | $CH_3$ | $CH_3$ | $CH_3$ | 2-$CH_3$-Ph | nD 1.5642 (18.3°C) |
| 309 | $CH_3$ | $CH_3$ | $CH_3$ | 3-$CH_3$-Ph | nD 1.5358 (19.4°C) |
| 310 | $CH_3$ | $CH_3$ | $CH_3$ | 4-$CH_3$-Ph | nD 1.5695 (19.1°C) |
| 311 | $CH_3$ | $CH_3$ | $CH_3$ | 3,4-$(CH_3)_2$-Ph | nD 1.5735 (19.6°C) |
| 312 | $CH_3$ | $CH_3$ | $CH_3$ | 2,5-$(CH_3)_2$-Ph | nD 1.5614 (22.4°C) |
| 313 | $CH_3$ | $CH_3$ | $CH_3$ | 3-$CH_3$-4-F-Ph | nD 1.5690 (22.8°C) |
| 314 | $CH_3$ | $CH_3$ | $CH_3$ | 2-F-4-Cl-5-$CH_3$-Ph | nD 1.5704 (22.7°C) |
| 315 | $CH_3$ | $CH_3$ | $CH_3$ | 2-$CH_3O$-Ph | nD 1.5529 (23.0°C) |
| 316 | $CH_3$ | $CH_3$ | $CH_3$ | 3-$CH_3O$-Ph | |
| 317 | $CH_3$ | $CH_3$ | $CH_3$ | 4-$CH_3O$-Ph | |
| 318 | $CH_3$ | $CH_3$ | $CH_3$ | 3-i-$C_4H_9O$-Ph | nD 1.5610 (18.7°C) |
| 319 | $CH_3$ | $CH_3$ | $CH_3$ | 3-s-$C_4H_9O$-Ph | nD 1.5589 (19.0°C) |

- Cont'd -

Table 3 (Cont'd)

| No. | $R^1$ | $R^2$ | $R^9$ | $R^{10}$ | Physical property |
|---|---|---|---|---|---|
| 320 | $CH_3$ | $CH_3$ | $CH_3$ | 3,4-$(CH_3O)_2$-Ph | m.p. 104.5-105.8°C |
| 321 | $CH_3$ | $CH_3$ | $CH_3$ | 2-$C_2H_5$-Ph | |
| 322 | $CH_3$ | $CH_3$ | $CH_3$ | 3-$C_2H_5$-Ph | |
| 323 | $CH_3$ | $CH_3$ | $CH_3$ | 4-$C_2H_5$-Ph | nD 1.5725 (17.2°C) |
| 324 | $CH_3$ | $CH_3$ | $CH_3$ | 4-i-$C_3H_7$-Ph | m.p. 82.9-87.0°C |
| 325 | $CH_3$ | $CH_3$ | $CH_3$ | 4-i-$C_4H_9$-Ph | m.p. 53.5-55.5°C |
| 326 | $CH_3$ | $CH_3$ | $CH_3$ | 2-$CF_3$-Ph | |
| 327 | $CH_3$ | $CH_3$ | $CH_3$ | 3-$CF_3$-Ph | nD 1.5420 (22.9°C) |
| 328 | $CH_3$ | $CH_3$ | $CH_3$ | 4-$CF_3$-Ph | |
| 329 | $CH_3$ | $CH_3$ | $CH_3$ | 2-$CF_3O$-Ph | |
| 330 | $CH_3$ | $CH_3$ | $CH_3$ | 3-$CF_3O$-Ph | nD 1.5354 (23.9°C) |
| 331 | $CH_3$ | $CH_3$ | $CH_3$ | 4-$CF_3O$-Ph | nD 1.5376 (16.8°C) |
| 332 | $CH_3$ | $CH_3$ | $CH_3$ | 2-$CHF_2O$-Ph | |
| 333 | $CH_3$ | $CH_3$ | $CH_3$ | 3-$CHF_2O$-Ph | nD 1.5562 (12.6°C) |
| 334 | $CH_3$ | $CH_3$ | $CH_3$ | 4-$CHF_2O$-Ph | |
| 335 | $CH_3$ | $CH_3$ | $CH_3$ | 2-$NO_2$-Ph | |
| 336 | $CH_3$ | $CH_3$ | $CH_3$ | 3-$NO_2$-Ph | m.p. 85.5°C |
| 337 | $CH_3$ | $CH_3$ | $CH_3$ | 4-$NO_2$-Ph | m.p. 83.8-88.1°C |
| 338 | $CH_3$ | $CH_3$ | $CH_3$ | 2,4,6-$(CH_3)_3$-Ph | nD 1.5630 (18.5°C) |
| 339 | $CH_3$ | $CH_3$ | $CH_3$ | 2,4,5-$(CH_3)_3$-Ph | |
| 340 | $CH_3$ | $CH_3$ | $CH_3$ | 2-CN-Ph | |
| 341 | $CH_3$ | $CH_3$ | $CH_3$ | 3-CN-Ph | nD 1.5665 (15.5°C) |

- Cont'd -

Table 3 (Cont'd)

| No. | $R^1$ | $R^2$ | $R^9$ | $R^{10}$ | Physical property |
|---|---|---|---|---|---|
| 342 | $CH_3$ | $CH_3$ | $CH_3$ | 4-CN-Ph | m.p. 97.1-98.6°C |
| 343 | $CH_3$ | $CH_3$ | $CH_3$ | 2-$CH_3$S-Ph | |
| 344 | $CH_3$ | $CH_3$ | $CH_3$ | 3-$CH_3$S-Ph | |
| 345 | $CH_3$ | $CH_3$ | $CH_3$ | 4-$CH_3$S-Ph | m.p. 100.7-101.5°C |
| 346 | $CH_3$ | $CH_3$ | $CH_3$ | 2-$CF_3$S-Ph | |
| 347 | $CH_3$ | $CH_3$ | $CH_3$ | 3-$CF_3$S-Ph | |
| 348 | $CH_3$ | $CH_3$ | $CH_3$ | 4-$CF_3$S-Ph | |
| 349 | $CH_3$ | $CH_3$ | $CH_3$ | 3-F-4-$CH_3$S-Ph | nD 1.5910 (19.0°C) |
| 350 | $CH_3$ | $CH_3$ | $CH_3$ | Pyridine-2-yl | nD 1.5622 (16.8°C) |
| 351 | $CH_3$ | $CH_3$ | $CH_3$ | Pyridine-3-yl | nD 1.5083 (17.2°C) |
| 352 | $CH_3$ | $CH_3$ | $CH_3$ | Pyridine-4-yl | nD 1.4990 (17.3°C) |
| 353 | $CH_3$ | $CH_3$ | $CH_3$ | Pyradine-2-yl | m.p. 148.3-149.2°C |
| 354 | $CH_3$ | $CH_3$ | $CH_3$ | Pyradine-3-yl | |
| 355 | $CH_3$ | $CH_3$ | $CH_3$ | Naphthalene-2-yl | nD 1.6022 (26.9°C) |
| 356 | $CH_3$ | $CH_3$ | $CH_3$ | Indane-5-yl | m.p. 91.7-93.6°C |
| 357 | $CH_3$ | $CH_3$ | $CH_3$ | Tetraline-6-yl | m.p. 91.6-95.0°C |
| 358 | $CH_3$ | $CH_3$ | $CH_3$ | Thiophene-2-yl | |
| 359 | $CH_3$ | $CH_3$ | $CH_3$ | 4-Br-Thiophene-2-yl | |
| 360 | $CH_3$ | $CH_3$ | $C_2H_5$ | Ph | |
| 361 | $CH_3$ | $CH_3$ | $C_2H_5$ | 2-F-Ph | |
| 362 | $CH_3$ | $CH_3$ | $C_2H_5$ | 3-F-Ph | |
| 363 | $CH_3$ | $CH_3$ | $C_2H_5$ | 4-F-Ph | nD 1.5613 (16.5°C) |

- Cont'd -

Table 3 (Cont'd)

| No. | $R^1$ | $R^2$ | $R^9$ | $R^{10}$ | Physical property |
|---|---|---|---|---|---|
| 364 | $CH_3$ | $CH_3$ | $C_2H_5$ | 2,4-$F_2$-Ph | nD 1.5467 (22.5°C) |
| 365 | $CH_3$ | $CH_3$ | $C_2H_5$ | 3,4-$F_2$-Ph | nD 1.5546 (14.3°C) |
| 366 | $CH_3$ | $CH_3$ | $C_2H_5$ | 2,4,5-$F_3$-Ph | |
| 367 | $CH_3$ | $CH_3$ | $C_2H_5$ | 2-Cl-Ph | |
| 368 | $CH_3$ | $CH_3$ | $C_2H_5$ | 3-Cl-Ph | |
| 369 | $CH_3$ | $CH_3$ | $C_2H_5$ | 4-Cl-Ph | m.p. 66.5-70.2°C |
| 370 | $CH_3$ | $CH_3$ | $C_2H_5$ | 2,4-$Cl_2$-Ph | |
| 371 | $CH_3$ | $CH_3$ | $C_2H_5$ | 3,4-$Cl_2$-Ph | m.p. 87.6-90.1°C |
| 372 | $CH_3$ | $CH_3$ | $C_2H_5$ | 2-$CH_3$-Ph | |
| 373 | $CH_3$ | $CH_3$ | $C_2H_5$ | 3-$CH_3$-Ph | |
| 374 | $CH_3$ | $CH_3$ | $C_2H_5$ | 4-$CH_3$-Ph | |
| 375 | $CH_3$ | $CH_3$ | $C_2H_5$ | 2,5-$(CH_3)_2$-Ph | nD 1.5480 (22.3°C) |
| 376 | $CH_3$ | $CH_3$ | $C_2H_5$ | 2-$CF_3$-Ph | |
| 377 | $CH_3$ | $CH_3$ | $C_2H_5$ | 3-$CF_3$-Ph | |
| 378 | $CH_3$ | $CH_3$ | $C_2H_5$ | 4-$CF_3$-Ph | |
| 379 | $CH_3$ | $CH_3$ | $C_2H_5$ | 2-$CH_3O$-Ph | |
| 380 | $CH_3$ | $CH_3$ | $C_2H_5$ | 3-$CH_3O$-Ph | |
| 381 | $CH_3$ | $CH_3$ | $C_2H_5$ | 4-$CH_3O$-Ph | |
| 382 | $CH_3$ | $CH_3$ | $C_2H_5$ | 2-$CF_3O$-Ph | |
| 383 | $CH_3$ | $CH_3$ | $C_2H_5$ | 3-$CF_3O$-Ph | |
| 384 | $CH_3$ | $CH_3$ | $C_2H_5$ | 4-$CF_3O$-Ph | |
| 385 | $CH_3$ | $CH_3$ | $C_2H_5$ | 2-CN-Ph | |

- Cont'd -

Table 3 (Cont'd)

| No. | $R^1$ | $R^2$ | $R^9$ | $R^{10}$ | Physical property |
|---|---|---|---|---|---|
| 386 | $CH_3$ | $CH_3$ | $C_2H_5$ | 3-CN-Ph | |
| 387 | $CH_3$ | $CH_3$ | $C_2H_5$ | 4-CN-Ph | |
| 388 | $CH_3$ | $CH_3$ | $C_2H_5$ | 2-$NO_2$-Ph | |
| 389 | $CH_3$ | $CH_3$ | $C_2H_5$ | 3-$NO_2$-Ph | |
| 390 | $CH_3$ | $CH_3$ | $C_2H_5$ | 4-$NO_2$-Ph | |
| 391 | $CH_3$ | $CH_3$ | $C_2H_5$ | 2-$CH_3$S-Ph | |
| 392 | $CH_3$ | $CH_3$ | $C_2H_5$ | 3-$CH_3$S-Ph | |
| 393 | $CH_3$ | $CH_3$ | $C_2H_5$ | 4-$CH_3$S-Ph | |
| 394 | $CH_3$ | $CH_3$ | $C_2H_5$ | 2-$CF_3$S-Ph | |
| 395 | $CH_3$ | $CH_3$ | $C_2H_5$ | 3-$CF_3$S-Ph | |
| 396 | $CH_3$ | $CH_3$ | $C_2H_5$ | 4-$CF_3$S-Ph | |
| 397 | $CH_3$ | $CH_3$ | $C_2H_5$ | Pyridine-2-yl | |
| 398 | $CH_3$ | $CH_3$ | $C_2H_5$ | Pyridine-3-yl | |
| 399 | $CH_3$ | $CH_3$ | $C_2H_5$ | Pyridine-4-yl | |
| 400 | $CH_3$ | $CH_3$ | $C_2H_5$ | Pyradine-2-yl | |
| 401 | $CH_3$ | $CH_3$ | $C_2H_5$ | Pyradine-3-yl | |
| 402 | $CH_3$ | $CH_3$ | $C_2H_5$ | Naphthalene-2-yl | |
| 403 | $CH_3$ | $CH_3$ | $C_2H_5$ | Indane-5-yl | |
| 404 | $CH_3$ | $CH_3$ | $C_2H_5$ | Tetraline-6-yl | |
| 405 | $CH_3$ | $CH_3$ | $C_2H_5$ | Thiophene-2-yl | |
| 406 | $CH_3$ | $CH_3$ | $C_2H_5$ | 4-Br-Thiophene-2-yl | |
| 407 | $C_2H_5$ | $CH_3$ | $CH_3$ | Ph | |

- Cont'd -

Table 3 (Cont'd)

| No. | $R^1$ | $R^2$ | $R^9$ | $R^{10}$ | Physical property |
|---|---|---|---|---|---|
| 408 | $C_2H_5$ | $CH_3$ | $CH_3$ | 2-F-Ph | |
| 409 | $C_2H_5$ | $CH_3$ | $CH_3$ | 3-F-Ph | |
| 410 | $C_2H_5$ | $CH_3$ | $CH_3$ | 4-F-Ph | nD 1.5578 (17.5°C) |
| 411 | $C_2H_5$ | $CH_3$ | $CH_3$ | 2,4-$F_2$-Ph | nD 1.5419 (22.4°C) |
| 412 | $C_2H_5$ | $CH_3$ | $CH_3$ | 3,4-$F_2$-Ph | nD 1.5530 (16.6°C) |
| 413 | $C_2H_5$ | $CH_3$ | $CH_3$ | 2,6-$F_2$-Ph | nD 1.5463 (14.0°C) |
| 414 | $C_2H_5$ | $CH_3$ | $CH_3$ | 2,4,5-$F_3$-Ph | |
| 415 | $C_2H_5$ | $CH_3$ | $CH_3$ | 2-Cl-Ph | |
| 416 | $C_2H_5$ | $CH_3$ | $CH_3$ | 3-Cl-Ph | nD 1.5777 (18.9°C) |
| 417 | $C_2H_5$ | $CH_3$ | $CH_3$ | 4-Cl-Ph | nD 1.5767 (15.8°C) |
| 418 | $C_2H_5$ | $CH_3$ | $CH_3$ | 2,4-$Cl_2$-Ph | paste |
| 419 | $C_2H_5$ | $CH_3$ | $CH_3$ | 3,4-$Cl_2$-Ph | |
| 420 | $C_2H_5$ | $CH_3$ | $CH_3$ | 2-$CH_3$-Ph | |
| 421 | $C_2H_5$ | $CH_3$ | $CH_3$ | 3-$CH_3$-Ph | nD 1.5644 (22.2°C) |
| 422 | $C_2H_5$ | $CH_3$ | $CH_3$ | 4-$CH_3$-Ph | |
| 423 | $C_2H_5$ | $CH_3$ | $CH_3$ | 2-$CH_3O$-Ph | nD 1.5529 (23.0°C) |
| 424 | $C_2H_5$ | $CH_3$ | $CH_3$ | 3-$CH_3O$-Ph | |
| 425 | $C_2H_5$ | $CH_3$ | $CH_3$ | 4-$CH_3O$-Ph | |
| 426 | $C_2H_5$ | $CH_3$ | $CH_3$ | 3,4-$(CH_3O)_2$-Ph | nD 1.5689 (17.6°C) |
| 427 | $C_2H_5$ | $CH_3$ | $CH_3$ | 2-$C_2H_5$-Ph | |
| 428 | $C_2H_5$ | $CH_3$ | $CH_3$ | 4-$C_2H_5$-Ph | m.p. 66.2-70.1°C |
| 429 | $C_2H_5$ | $CH_3$ | $CH_3$ | 2-$NO_2$-Ph | |

- Cont'd -

Table 3 (Cont'd)

| No. | $R^1$ | $R^2$ | $R^9$ | $R^{10}$ | Physical property |
|---|---|---|---|---|---|
| 430 | $C_2H_5$ | $CH_3$ | $CH_3$ | 3-$NO_2$-Ph | |
| 431 | $C_2H_5$ | $CH_3$ | $CH_3$ | 4-$NO_2$-Ph | |
| 432 | $C_2H_5$ | $CH_3$ | $CH_3$ | 2-CN-Ph | nD 1.5862 (16.2°C) |
| 433 | $C_2H_5$ | $CH_3$ | $CH_3$ | 3-CN-Ph | |
| 434 | $C_2H_5$ | $CH_3$ | $CH_3$ | 4-CN-Ph | |
| 435 | $C_2H_5$ | $CH_3$ | $CH_3$ | 2-$CF_3$-Ph | |
| 436 | $C_2H_5$ | $CH_3$ | $CH_3$ | 3-$CF_3$-Ph | |
| 437 | $C_2H_5$ | $CH_3$ | $CH_3$ | 4-$CF_3$-Ph | |
| 438 | $C_2H_5$ | $CH_3$ | $CH_3$ | 2-$CHF_2O$-Ph | |
| 439 | $C_2H_5$ | $CH_3$ | $CH_3$ | 3-$CHF_2O$-Ph | |
| 440 | $C_2H_5$ | $CH_3$ | $CH_3$ | 4-$CF_3O$-Ph | m.p. 87.3-88.9°C |
| 441 | $C_2H_5$ | $CH_3$ | $CH_3$ | 2-$CH_3S$-Ph | |
| 442 | $C_2H_5$ | $CH_3$ | $CH_3$ | 3-$CH_3S$-Ph | |
| 443 | $C_2H_5$ | $CH_3$ | $CH_3$ | 4-$CH_3S$-Ph | m.p. 104.6-106.5°C |
| 444 | $C_2H_5$ | $CH_3$ | $CH_3$ | 2-$CF_3S$-Ph | |
| 445 | $C_2H_5$ | $CH_3$ | $CH_3$ | 3-$CF_3S$-Ph | |
| 446 | $C_2H_5$ | $CH_3$ | $CH_3$ | 4-$CF_3S$-Ph | |
| 447 | $C_2H_5$ | $CH_3$ | $CH_3$ | Pyridine-2-yl | nD 1.5618 (21.2°C) |
| 448 | $C_2H_5$ | $CH_3$ | $CH_3$ | Pyridine-3-yl | |
| 449 | $C_2H_5$ | $CH_3$ | $CH_3$ | Pyridine-4-yl | |
| 450 | $C_2H_5$ | $CH_3$ | $CH_3$ | Pyradine-2-yl | nD 1.5618 (21.2°C) |
| 451 | $C_2H_5$ | $CH_3$ | $CH_3$ | Pyradine-3-yl | |

- Cont'd -

Table 3 (Cont'd)

| No. | $R^1$ | $R^2$ | $R^9$ | $R^{10}$ | Physical property |
|---|---|---|---|---|---|
| 452 | $C_2H_5$ | $CH_3$ | $CH_3$ | Naphthalene-2-yl | |
| 453 | $C_2H_5$ | $CH_3$ | $CH_3$ | Indane-5-yl | nD 1.5768 (23.1°C) |
| 454 | $C_2H_5$ | $CH_3$ | $CH_3$ | Tetraline-6-yl | nD 1.5757 (23.2°C) |
| 455 | $C_2H_5$ | $CH_3$ | $CH_3$ | Thiophene-2-yl | |
| 456 | $C_2H_5$ | $CH_3$ | $CH_3$ | 4-Br-Thiophene-2-yl | nD 1.5618 (21.2°C) |
| 457 | $C_2H_5$ | $CH_3$ | $CH_3$ | Ph | |
| 458 | $CH_3$ | $C_2H_5$ | $CH_3$ | 2-F-Ph | |
| 459 | $CH_3$ | $C_2H_5$ | $CH_3$ | 3-F-Ph | |
| 460 | $CH_3$ | $C_2H_5$ | $CH_3$ | 4-F-Ph | |
| 461 | $CH_3$ | $C_2H_5$ | $CH_3$ | 2,4-$F_2$-Ph | |
| 462 | $CH_3$ | $C_2H_5$ | $CH_3$ | 3,4-$F_2$-Ph | |
| 463 | $CH_3$ | $C_2H_5$ | $CH_3$ | 2,6-$F_2$-Ph | |
| 464 | $CH_3$ | $C_2H_5$ | $CH_3$ | 2,4,5-$F_3$-Ph | |
| 465 | $CH_3$ | $C_2H_5$ | $CH_3$ | 2-Cl-Ph | |
| 466 | $CH_3$ | $C_2H_5$ | $CH_3$ | 3-Cl-Ph | |
| 467 | $CH_3$ | $C_2H_5$ | $CH_3$ | 4-Cl-Ph | |
| 468 | $CH_3$ | $C_2H_5$ | $CH_3$ | 2,4-$Cl_2$-Ph | |
| 469 | $CH_3$ | $C_2H_5$ | $CH_3$ | 3,4-$Cl_2$-Ph | |
| 470 | $CH_3$ | $C_2H_5$ | $CH_3$ | 2-$CH_3$-Ph | |
| 471 | $CH_3$ | $C_2H_5$ | $CH_3$ | 3-$CH_3$-Ph | |
| 472 | $CH_3$ | $C_2H_5$ | $CH_3$ | 4-$CH_3$-Ph | |
| 473 | $CH_3$ | $C_2H_5$ | $CH_3$ | 2-$CH_3O$-Ph | |

- Cont'd -

Table 3 (Cont'd)

| No. | $R^1$ | $R^2$ | $R^9$ | $R^{10}$ | Physical property |
|---|---|---|---|---|---|
| 474 | $CH_3$ | $C_2H_5$ | $CH_3$ | 3-$CH_3O$-Ph | |
| 475 | $CH_3$ | $C_2H_5$ | $CH_3$ | 4-$CH_3O$-Ph | |
| 476 | $CH_3$ | $C_2H_5$ | $CH_3$ | 3,4-$(CH_3O)_2$-Ph | |
| 477 | $CH_3$ | $C_2H_5$ | $CH_3$ | 2-$C_2H_5$-Ph | |
| 478 | $CH_3$ | $C_2H_5$ | $CH_3$ | 4-$C_2H_5$-Ph | |
| 479 | $CH_3$ | $C_2H_5$ | $CH_3$ | 2-$NO_2$-Ph | |
| 480 | $CH_3$ | $C_2H_5$ | $CH_3$ | 3-$NO_2$-Ph | |
| 481 | $CH_3$ | $C_2H_5$ | $CH_3$ | 4-$NO_2$-Ph | |
| 482 | $CH_3$ | $C_2H_5$ | $CH_3$ | 2-CN-Ph | |
| 483 | $CH_3$ | $C_2H_5$ | $CH_3$ | 3-CN-Ph | |
| 484 | $CH_3$ | $C_2H_5$ | $CH_3$ | 4-CN-Ph | |
| 485 | $CH_3$ | $C_2H_5$ | $CH_3$ | 2-$CF_3$-Ph | |
| 486 | $CH_3$ | $C_2H_5$ | $CH_3$ | 3-$CF_3$-Ph | |
| 487 | $CH_3$ | $C_2H_5$ | $CH_3$ | 4-$CF_3$-Ph | |
| 488 | $CH_3$ | $C_2H_5$ | $CH_3$ | 2-$CHF_2O$-Ph | |
| 489 | $CH_3$ | $C_2H_5$ | $CH_3$ | 3-$CHF_2O$-Ph | |
| 490 | $CH_3$ | $C_2H_5$ | $CH_3$ | 4-$CHF_2O$-Ph | |
| 491 | $CH_3$ | $C_2H_5$ | $CH_3$ | 2-$CH_3S$-Ph | |
| 492 | $CH_3$ | $C_2H_5$ | $CH_3$ | 3-$CH_3S$-Ph | |
| 493 | $CH_3$ | $C_2H_5$ | $CH_3$ | 4-$CH_3S$-Ph | |
| 494 | $CH_3$ | $C_2H_5$ | $CH_3$ | 2-$CF_3S$-Ph | |
| 495 | $CH_3$ | $C_2H_5$ | $CH_3$ | 3-$CF_3S$-Ph | |
| 496 | $CH_3$ | $C_2H_5$ | $CH_3$ | 4-$CF_3S$-Ph | |

Table 3 (Cont'd)

| No. | $R^1$ | $R^2$ | $R^9$ | $R^{10}$ | Physical property |
|---|---|---|---|---|---|
| 497 | $CH_3$ | $CH_3$ | $CH_3$ | 3-$PhCH_2O$-Ph | nD 1.5166 (19.0°C) |
| 498 | $CH_3$ | $CH_3$ | $CH_3$ | 3-I-Ph | nD 1.6092 (10.4°C) |
| 499 | $CH_3$ | $CH_3$ | $CH_3$ | 3-Br-4-F-Ph | nD 1.5739 (12.6°C) |
| 500 | $CH_3$ | $CH_3$ | $CH_3$ | 4-Cl-3-$CH_3$-Ph | nD 1.5828 (12.6°C) |
| 501 | $CH_3$ | $CH_3$ | $C_2H_5$ | 4-F-3-$CH_3$-Ph | m.p. 78.1-80.7°C |
| 502 | $CH_3$ | $CH_3$ | $C_2H_5$ | 3,4-$(CH_3)_2$-Ph | m.p. 92.6-98.9°C |
| 503 | $CH_3$ | $CH_3$ | $CH_3$ | 4-$N(CH_3)_2$-Ph | nD 1.5973 (19.5°C) |
| 504 | $CH_3$ | $CH_3$ | $CH_3$ | 4-Cl-3-$NO_2$-Ph | m.p. 101.6-103.3°C |
| 505 | $C_2H_5$ | $CH_3$ | $CH_3$ | 3-Br-Ph | paste |
| 506 | $C_2H_5$ | $CH_3$ | $CH_3$ | 3-$CF_3O$-Ph | paste |

Table 4 shows NMR datas of compound No. 188, 505 and 506 listed in Table 2 and Table 3.

Table 4

| | $^1$H-NMR[$CDCl_3$/TMS, ppm 200 MHz] |
|---|---|
| 188 | 2.48 (s, 3H), 3.74 (s, 3H), 3.77 (2, 3H), 4.39 (s, 2H), 6.66-6.67 (d, 1H), 7.00-7.02 (d, 1H), 6.96-6.97 (d, 1H), 7.31-7.38 (m, 3H), 7.47-7.56 (bm, 1H). |
| 505 | 1.24 (t, 3H, J=7Hz), 2.21 (s, 3H), 2.29 (s, 3H), 3.80 (s, 3H), 3.99 (q, 2H, J=7Hz), 5.33 (s, 2H), 7.23-7.31 (m, 1H), 7.32-7.42 (m, 4H), 7.47-7.58 (m, 1H), 7.73-7.78 (m, 1H), 8.00 (t, 1H, J=1.9Hz). |
| 506 | 1.24 (t, 3H, J=7Hz), 2.22 (s, 3H), 2.32 (s, 3H), 3.79 (s, 3H), 3.99 (q, 2H, J=7Hz), 5.34 (s, 2H), 7.23-7.26 (m, 1H), 7.45-7.53 (m, 4H), 7.51-7.61 (m, 1H), 7.70-7.80 (m, 2H). |

The compound of the general formula (III), i.e., the starting compound for producing the N-substituted phenylcarbamic acid derivative of the general formula (I) of the present invention can be produced, for example, by the process illustrated below.

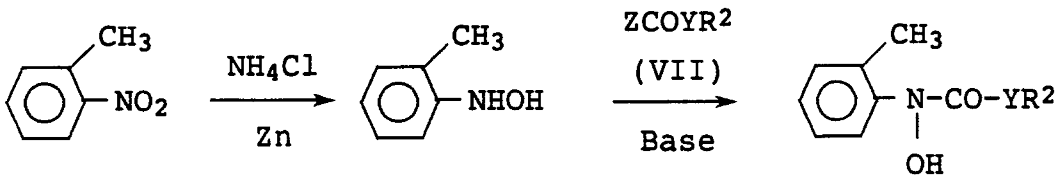


(IX) (VIII) (VI)

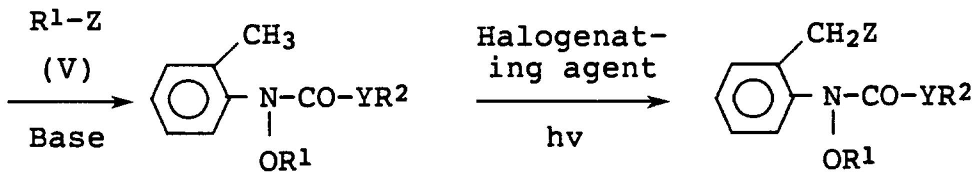


(IV) (III)

wherein $R^1$, $R^2$, Z and Y are as defined above.

o-Nitrotoluene of the structural formula (IX) is reacted with ammonium chloride in the presence of zinc to prepare N-2-methylphenylhydroxyamine of the structural formula (VIII) (Organic Syntheses Collective Volume III, p. 668, 1955). The hydroxyamine is reacted with a compound of the general formula (VII) in the presence of a base after or without isolation of the hydroxyamine to obtain a compound of the general formula (VI). This compound (VI) is reacted with a compound of the general formula (V) in the presence of a base after or without isolation of the compound (VI) to obtain a compound of the general formula (IV). This compound (IV) is reacted with a halogenating agent under light irradiation after or without isolation of the compound (IV), whereby the compound of the general formula (III) can be produced.

Typical examples of the present invention are described below, but they should not be construed as limiting the scope of the invention.

Example 1

1-1. Production of N-2-methylphenylhydroxyamine

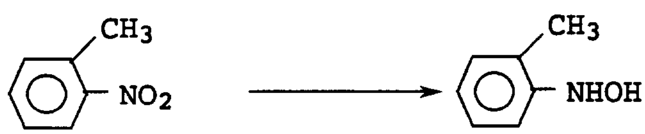

To 400 ml of ethanol were added 70 g (0.51 mole) of o-nitrotoluene and 66.8 g of zinc, and 300 ml of an aqueous solution of 30 g of ammonium chloride was slowly dropped into the resulting mixture with stirring at a reaction temperature of 45 - 55 °C.

After completion of the dropping, the reaction mixture was filtered at room temperature and the filtrate was concentrated under reduced pressure. The resulting residue was added to 300 ml of water and the desired compound was extracted with ethyl acetate. The extracted solution was washed with a saturated aqueous sodium chloride solution and dried over sodium sulfate, after which the solvent was distilled off under reduced pressure to obtain 47 g (yield 74%) of the desired compound.

The N-2-methylphenylhydroxyamine obtained was used in the subsequent reaction without purification.

1-2. Production of methyl N-hydroxy-N-2-methylphenylcarbamate

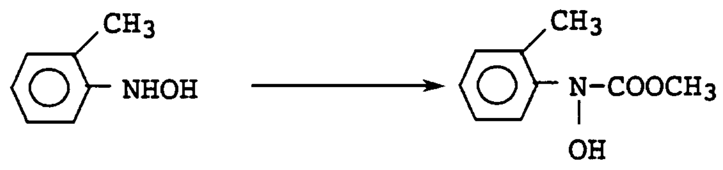

To 60 ml of tetrahydrofuran were added 5.9 g (0.048 mole) of the N-2-methylphenylhydroxyamine obtained in 1-1 and 3.9 g of triethylamine, and 40 ml of a solution of 3.6 g of methyl chlorocarbonate in tetrahydrofuran was added dropwise at 0 °C over a period of 40 minutes. The reaction was carried out with stirring at 0 °C for another 30 minutes.

After completion of the reaction, the reaction mixture was filtered at room temperature. The resulting filtrate was poured into 40 ml of water and the desired compound was extracted with ethyl acetate. The extracted solution was washed with a saturated aqueous sodium chloride solution and dried over sodium sulfate, after which the solvent was distilled off under reduced pressure. The resulting residue was purified by a silica gel column chromatography to obtain 6.0 g of the desired compound.

Physical property:

NMR [$CDCl_3$/TMS, δ values (ppm)]

2.35 (s, 3H), 3.74 (S, 3H), 7.20-7.30 (m, 3H), 7.24-7.29 (m, 1H), 9.20-9.27 (br, 1H).

Yield: 69%.

1-3. Production of methyl N-ethoxy-N-2-methylphenylcarbamate

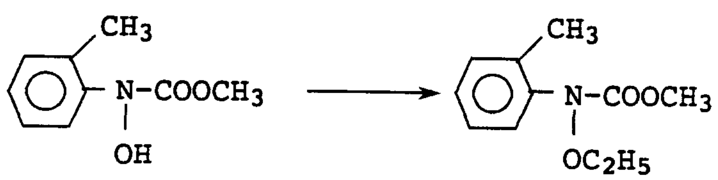

To 30 ml of acetone were added 2.25 g (0.012 mole) of the methyl N-hydroxy-N-2-methylphenylcarbamate obtained in 1-2, 2.1 g of anhydrous potassium carbonate and 1.6 g of ethyl bromide, and the reaction was carried out with heating under reflux for 3 hours.

After completion of the reaction, the reaction mixture was poured into 40 ml of water and the desired compound was extracted with ethyl acetate. The extracted solution was washed with a saturated aqueous sodium chloride solution and dried over sodium sulfate, after which the solvent was distilled off under reduced pressure. The resulting residue was purified by a silica gel column chromatography to obtain 1.6 g of the desired compound.

Physical property:

NMR [$CDCl_3$/TMS, δ values (ppm)]

1.23 (t, 3H, J = 7.2Hz), 3.77 (S, 3H), 3.96 (q, 2H, J = 7.2Hz), 7.19-7.32 (m, 4H).

Yield: 62%.

Methyl N-2-methylphenyl-N-methoxycarbamate was produced in a similar manner as above.

Physical property:

NMR [$CDCl_3$/TMS, δ values (ppm)]

2.29 (s, 3H), 3.73 (s, 3H), 3.78 (s, 3H), 7.20-7.32 (m, 4H).

1-4. Production of methyl N-2-bromomethylphenyl-N-ethoxycarbamate

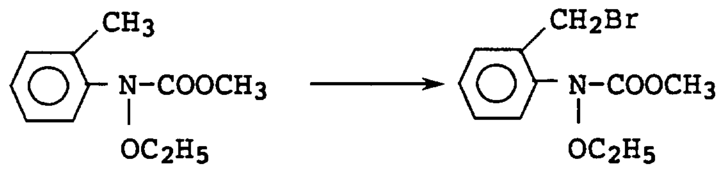

To 20 ml of carbon tetrachloride were added 1.6 g (0.076 mole) of the methyl N-ethoxy-N-2-methylphenylcarbamate, 1.4 g of N-bromosuccinimide and a small amount of azobisbutyronitrile, and the reaction was carried out with heating under reflux for 5 hours under light irradiation (TOKI REFLIGHT. Model LC-107).

After completion of the reaction, 30 ml of water was added to the reaction mixture, and the organic layer was separated, washed with a saturated aqueous sodium chloride solution and dried over sodium sulfate. Then, the solvent was distilled off under reduced pressure and the resulting residue was purified by a silica gel column chromatography to obtain 1.5 g of the desired compound.

Physical property: nD 1.5460 (18.5 ° C).

Yield: 68%.

Methyl N-2-bromomethylphenyl-N-methoxycarbamate was produced in a similar manner as above.

Physical property: nD 1.5570 (18.5 ° C).

1-5. Production of methyl N-2-[1-acetyl-2-[1-(4-chlorophenyl)ethylidene]-1-hydrazinomethyl]phenyl-N-ethoxycarbamate (compound No. 417)

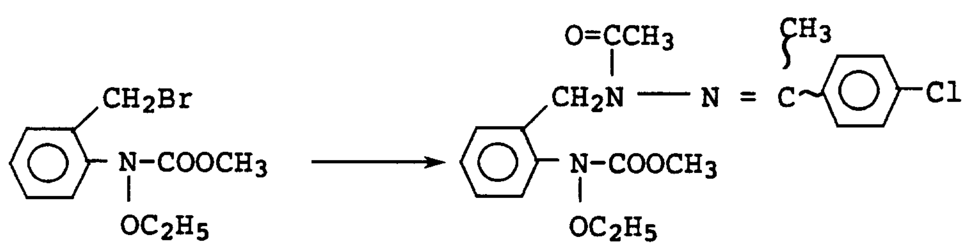

In 20 ml of N,N-dimethylformamide was suspended 0.11 g of sodium hydride (62.5% in oil), after which 0.57 g of 1-acetyl-2-[1-(4-chlorophenyl)-ethylidene]hydrazine was added to the suspension and the resulting mixture was stirred at room temperature for 10 minutes. Then, 5 ml of a solution of 0.4 g of methyl N-(2-bromomethylphenyl)-N-ethoxycarbamate in N,N-dimethylformamide was slowly dropped into the mixture, and the reaction was carried out at room temperature.

After completion of the reaction, the reaction mixture was poured into 50 ml of water and the desired compound was extracted with ethyl acetate. The extracted solution was washed with a saturated aqueous sodium chloride solution and dried over sodium sulfate, after which the solvent was distilled off under reduced pressure. The resulting residue was purified by a silica gel column chromatography to obtain 0.48 g of the desired compound.

Physical property: nD 1.5767 (15.8 ° C).

Yield: 50%.

Example 2

Production of methyl N-methoxy-N-2-[[(4-methoxyphenylimino)(methylthio)methyl]thiomethyl]-phenylcarbamate (compound No. 184)

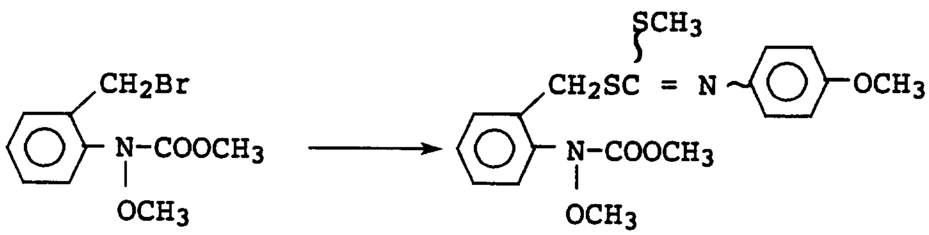

In 20 ml of dried tetrahydrofuran was suspended 0.06 g of sodium hydride (62.5% in oil), after which 0.34 g of methyl 4-methoxyphenyldithiocarbamate was added to the suspension and the resulting mixture was stirred at room temperature for 10 minutes. Then, 5 ml of a solution of 0.4 g of methyl N-(2-bromomethylphenyl)-N-ethoxycarbamatein tetrahydrofuran was slowly dropped into the mixture, and the reaction was carried out at room temperature.

After completion of the reaction, the reaction mixture was poured into 30 ml of water and the desired compound was extracted with ethyl acetate. The extracted solution was washed with a saturated aqueous sodium chloride solution and dried over sodium sulfate, after which the solvent was distilled off under reduced pressure. The resulting residue was purified by a silica gel column chromatography to obtain 0.28 g of the desired compound.

Physical property: nD 1.6129 (21.6 ° C).

Yield: 47%.

Example 3

Production of Methyl N-methoxy-N-2-(α-methylthio-4-chlorobenzylideneaminooxymethyl)phenyl carbamate (compound No. 7)

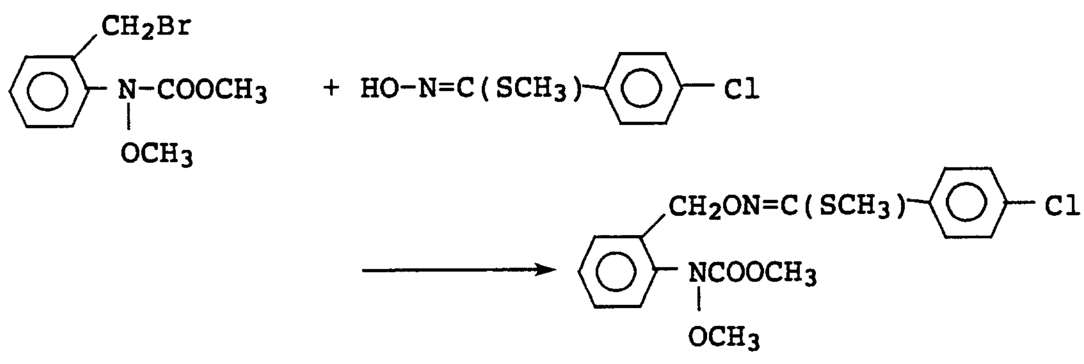

In 30 ml of N,N-dimethylformamide was dissolved 0.31 g (0.015 mole) of 4-chlorobenzohydroxamyl methyl thioether, after which 0.1 g (0.015 mole) of potassium hydroxide powder was added to the solution and the resulting mixture was stirred at room temperature for 10 minutes. Then, 0.4 g (0.015 mole) of methyl N-2-bromomethylphenyl-N-methoxycarbamate was added to the mixture and the reaction was carried out at room temperature for 3 hours.

After completion of the reaction, the reaction mixture containing the desired compound was poured into ice water and the desired compound was extracted with ethyl acetate. The extracted solution was washed with water and an aqueous sodium chloride solution, dried over anhydrous sodium sulfate, and then concentrated under reduced pressure. The resulting residue was purified by a silica gel dry column chromatography to obtain 0.25 g of the desired compound.

Physical property: nD 1.5795 (20.8 ° C).

Yield: 43%.

The N-substituted phenylcarbamic acid derivatives of the general formula (I) of the present invention are useful as agricultural and horticultural fungicides and are very effective in controlling various diseases, for example, rice blast (Pyricularia oryzae), rice sheath blight (Rhizoctonia solani), rice helminthosporium leaf spot (Cochiobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), apple scab (Venturia inaequalis), apple alternaria leaf spot (Alternaria mali), pear black spot (Alternaria kikuchiana), and citrus melanose (Diaporthe citri).

The agricultural and horticultural fungicide of the present invention has a marked fungicidal effect on the above-exemplified diseases which damage paddy field crops, upland crops, fruit trees, vegetables, other crops, flowers and ornamental plants, and the like. Therefore, the desired effects of the agricultural and horticultural fungicide of the present invention can be obtained by applying the fungicide to the paddy field water, stalks and leaves of fruit trees, vegetables, other crops, flowers and ornamental plants, soil, etc., at a season at which the diseases are expected to occur, before their occurrence or at the time when their occurrence is confirmed.

When the N-substituted phenylcarbamic acid derivative of the general formula (I) of the present invention is used as an agricultural and horticultural fungicide, it is generally prepared into conveniently usable forms according to an ordinary manner for preparation of agrochemicals.

That is, the N-substituted phenylcarbamic acid derivative of the general formula (I) of the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The agricultural and horticultural fungicide containing the N-substituted phenylcarbamic acid derivative of the general formula (I) of the present invention as an active ingredient is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases.

The applying dosage of the agricultural and horticultural fungicide containing the N-substituted phenylcarbamic acid derivative of the general formula (I) of the present invention as an active ingredient is varied depending upon various factors such as a purpose, diseases to be controlled, a growth state of a plant, tendency of disease occurrence, weather, environmental conditions, a preparation form, an application method, an application site and application time. It may be properly chosen in the range of 0.1 g to 1 kg (in terms of the active ingredient) per 10 ares depending upon purposes.

The agricultural and horticultural fungicide containing the N-substituted phenylcarbamic acid derivative of the general formula (I) of the present invention as an active ingredient may be used in admixture with other agricultural and horticultural fungicides in order to expand both spectrum of controllable diseases and the period of time when effective applications are possible or to reduce the dosage.

Typical preparation examples and test examples of the present invention are described below but they should not be construed as limiting the scope of the invention.

In the preparation examples, parts are all by weight.

| Formulation Example 1 | |
|---|---|
| Each compound of the invention | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

| Formulation Example 2 | |
|---|---|
| Each compound of the invention | 3 parts |
| Clay powder | 82 parts |
| Diatomaceous earth powder | 15 parts |

A dust was prepared by mixing uniformly and grinding the above ingredients.

| Formulation Example 3 | |
|---|---|
| Each compound of the invention | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

| Formulation Example 4 | |
|---|---|
| Each compound of the invention | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

Test Example 1

Controlling effect on barley powdery mildew

Potted barley plants at the 1 leaf stage were inoculated with spores of powdery mildew fungus (Erysiphe graminis f. sp. hordei) by sprinkling. After 24 hours, they were sprayed with a 200 ppm liquid chemical containing each compound of the present invention as active ingredient, and then allowed to stand in a room thermostated at 25 °C.

After one week of the inoculation, the lesion area of each leaf was measured and then compared with that on the untreated plot, whereby the effect was judged according to the following criterion.

| Effect | Controlling degree (%) |
|---|---|
| A | 100 - 95 |
| B | 94 - 80 |
| C | 79 - 60 |
| D | 59 - 0 |

The results obtained are shown in Table 5.

Test Example 2

Controlling effect on cucumber downy mildew

Potted cucumber plants at the 2 leaf stage were sprayed with a 200 ppm liquid chemical containing each compound of the present invention as active ingredient. After 24 hours, they were inoculated with a suspension of zoospores of downy mildew fungus (Pseudoperonospora cubensis) by spraying.

After the inoculation, the plants were placed in a moist chamber at 25 °C for 1 day and then a greenhouse for 6 days to cause the disease sufficiently. Thereafter, the degree of lesions of each leaf was investigated and the effect was judged according to the same criterion as described in Test Example 1.

The results obtained are shown in Table 5.

Test Example 3

Controlling effect on rice blast by spraying

Potted rice plants (cultivar: Nihonbare) at the 5 leaf stage were sufficiently sprayed with a 200 ppm liquid chemical containing each compound of the present invention as active ingredient. Twenty-four hours after the spraying, the plants were inoculated with a suspension of spores of blast fungus (Pyricularia oryzae) by spraying.

After the inoculation, the plants were placed in a moist chamber for 1 day and then a greenhouse for 6 days to cause the disease sufficiently. Then, lesions in each leaf were counted and then compared with those on the untreated plot. The controlling degree was calculated and the effect was judged according to the same criterion as described in Test Example 1.

The results obtained are shown in Table 5.

Test Example 4

Controlling effect on tomato late blight

Potted tomato plants at the 4 leaf stage were sprayed with a 200 ppm liquid chemical containing each compound of the present invention as active ingredient. After 24 hours, they were inoculated with a suspension of zoospores of late blight fungus (Phytophthora infestans) by spraying. The plants were placed in a moist chamber at 25°C for 1 day and then a greenhouse for 6 days to cause the disease sufficiently. Thereafter, the degree of lesions of each leaf was investigated and then compared with that on the untreated plot, whereby the effect was judged according to the same criterion as described in Test Example 1.

The results obtained are shown in Table 5.

Test Example 5

Controlling effect (curative effect) on cucumber gray mold

The cotyledons of potted cucumber plants at the 1 leaf stage were cut off and each of them was inoculated with a mycelial tuft of gray mold fungus (Botrytis cinerea) cultured on PSA medium. After having been placed in a moist chamber at 15°C for 24 hours, the cotyledons were immersed in a 200 ppm liquid chemical containing each compound of the present invention as active ingredient.

Then, the cotyledons were placed in a moist chamber at 15°C for 3 days to cause the disease sufficiently, after which the diameter of lesions was measured and then compared with that on the untreated plot, whereby the effect was judged according to the same criterion as described in Test Example 1.

The results obtained are shown in Table 5.

Test Example 6

Controlling effect (preventive effect) on cucumber gray mold

Potted cucumber plants at the 1 leaf stage were sprayed with a 200 ppm liquid chemical containing each compound of the present invention as active ingredient. After 24 hours, the cotyledons of the plants were cut off and each of them was inoculated with a mycelial tuft of gray mold fungus (Botrytis cinerea) cultured on PSA medium.

After the inoculation, the cotyledons were placed in a moist chamber at 15°C for 5 days to cause the disease sufficiently, after which the diameter of lesions was measured and then compared with that on the untreated plot, whereby the effect was judged according to the same criterion as described in Test Example 1.

The results obtained are shown in Table 5.

Table 5

| No. | Erg | Psc | Pyo | Phi | Boc curing | Boc Prevention |
|---|---|---|---|---|---|---|
| 2 | A | A | A | C | A | - |
| 4 | A | A | A | - | A | - |
| 7 | A | A | A | A | A | B |
| 8 | A | A | A | C | B | - |
| 10 | A | A | A | A | B | B |
| 13 | A | A | A | A | A | - |
| 15 | A | A | A | C | A | - |
| 16 | A | A | A | A | A | B |
| 23 | A | A | A | A | A | A |
| 25 | - | A | B | - | - | - |
| 27 | A | A | C | B | A | A |
| 31 | A | A | - | C | - | - |
| 32 | A | - | A | B | C | A |
| 154 | A | - | A | - | A | - |
| 165 | A | A | A | B | A | - |
| 169 | A | A | A | B | - | - |
| 175 | A | B | A | - | - | C |
| 178 | B | A | A | B | - | - |
| 184 | - | A | A | B | A | - |
| 187 | A | A | A | B | - | - |
| 188 | - | - | A | B | - | - |
| 190 | A | A | A | A | C | - |
| 191 | A | - | - | - | - | - |

-Cont'd -

Table 5 (Cont'd)

| No. | Erg | Psc | Pyo | Phi | Boc curing | Boc Prevention |
|---|---|---|---|---|---|---|
| 192 | A | C | A | C | - | - |
| 193 | A | A | A | B | A | - |
| 194 | - | A | - | B | A | A |
| 284 | A | A | A | B | A | A |
| 285 | A | A | A | B | A | A |
| 286 | A | A | A | A | A | A |
| 289 | A | A | A | A | A | A |
| 290 | A | A | A | A | A | A |
| 291 | A | A | A | B | A | A |
| 295 | A | A | A | B | A | A |
| 296 | A | A | A | B | A | A |
| 301 | A | A | B | B | C | A |
| 306 | A | A | A | B | A | B |
| 307 | A | A | A | A | A | A |
| 308 | A | B | A | B | A | A |
| 309 | A | A | A | B | A | A |
| 310 | A | A | A | A | A | A |
| 311 | A | B | A | B | B | A |
| 312 | A | A | A | B | A | A |
| 313 | A | A | A | B | A | A |
| 314 | A | A | A | B | A | B |
| 318 | A | C | A | C | A | - |
| 319 | A | C | A | C | A | C |
| 320 | A | A | A | - | A | C |
| 323 | A | B | A | - | - | C |

-Cont'd -

Table 5 (Cont'd)

| No. | Erg | Psc | Pyo | Phi | Boc curing | Boc Prevention |
|---|---|---|---|---|---|---|
| 324 | A | A | A | - | A | - |
| 325 | A | A | - | A | - | A |
| 327 | A | A | A | B | A | A |
| 330 | A | A | A | B | A | A |
| 331 | A | A | A | C | - | B |
| 333 | A | A | A | A | - | A |
| 336 | A | A | A | A | A | A |
| 337 | A | A | A | C | B | C |
| 338 | A | - | A | B | A | A |
| 342 | B | A | A | B | A | C |
| 345 | A | A | A | - | A | C |
| 349 | A | - | - | - | C | - |
| 350 | A | - | A | - | A | B |
| 351 | - | - | A | B | A | A |
| 352 | - | A | B | C | A | B |
| 353 | A | C | A | - | A | - |
| 355 | A | A | A | A | A | B |
| 356 | A | B | A | B | - | A |
| 357 | A | C | A | B | - | A |
| 363 | A | A | A | B | A | A |
| 364 | A | A | A | B | A | C |
| 365 | A | A | A | B | A | B |
| 369 | A | A | A | A | A | A |
| 371 | A | A | A | - | B | - |
| 375 | A | B | C | - | - | - |

-Cont'd -

Table 5 (Cont'd)

| No. | Erg | Psc | Pyo | Phi | Boc curing | Boc Prevention |
|---|---|---|---|---|---|---|
| 410 | A | A | A | A | A | A |
| 411 | A | - | A | - | A | B |
| 412 | A | - | A | A | C | - |
| 413 | A | A | A | B | A | A |
| 416 | A | A | A | A | A | C |
| 417 | A | A | A | B | A | A |
| 421 | A | A | A | - | A | A |
| 426 | A | C | B | - | B | C |
| 428 | A | B | B | - | - | - |
| 431 | A | - | - | - | - | - |
| 440 | A | A | C | - | B | C |
| 443 | B | B | C | - | - | - |
| 447 | A | B | A | - | A | B |
| 450 | A | A | A | C | A | B |
| 453 | A | B | B | - | - | - |
| 454 | A | - | - | C | - | - |
| 456 | A | - | A | - | - | A |
| 497 | A | A | A | A | B | B |
| 498 | A | A | A | C | A | A |

NOTE:
- Erg barley powdery mildew
- Psc cucumber downy mildew
- Pyo rice blast
- Phi tomato late blight
- Boc cucumber gray mold

## Claims

**1.** An N-substituted phenylcarbamic acid derivative represented by the general formula (I):

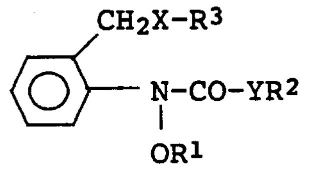

(I)

wherein $R^1$ is a hydrogen atom, a $C_{1-6}$alkyl group, a halo-$C_{1-6}$alkyl group, a $C_{1-6}$alkoxy-$C_{1-6}$alkyl group, a $C_{2-6}$alkenyl group, a $C_{2-6}$alkynyl group or a halo-$C_{1-6}$alkoxy-$C_{1-6}$alkyl group, $R^2$ is a hydrogen atom, a $C_{1-6}$alkyl group, a halo-$C_{1-6}$alkyl group, a $C_{1-6}$alkoxy-$C_{1-6}$alkyl group, a $C_{2-6}$alkenyl group, a $C_{2-6}$alkynyl group, a halo-$C_{1-6}$alkoxy-$C_{1-6}$alkyl group or a cyano-$C_{1-6}$alkyl group, X is an oxygen atom, a sulfur atom or a group represented by the formula:

$N(R^8)$

(wherein $R^8$ is a formyl group, a $C_{1-6}$alkyl group, a halo-$C_{1-6}$alkyl group, a $C_{1-6}$alkylcarbonyl group, a halo-$C_{1-6}$alkylcarbonyl group, a $C_{1-6}$alkoxycarbonyl group, a $C_{1-6}$alkoxy-$C_{1-6}$alkylcarbonyl group or a $C_{1-6}$alkylsulfonyl group), $R^3$ is as follows:

in the case of X being an oxygen atom, $R^3$ is a group represented by the formula:

$-N=C(R^4)R^5$

(wherein $R^4$ and $R^5$ are as follows:

in the case of $R^4$ being a $C_{1-6}$alkylthio group, $R^5$ is a hydrogen atom; a cyano group; a $C_{1-6}$alkyl group; a halo-$C_{1-6}$alkyl group; a $C_{3-6}$cycloalkyl group; a halo-$C_{3-6}$cycloalkyl group; a $C_{3-6}$cycloalkyl-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; a $C_{1-6}$alkoxy-$C_{1-6}$alkyl group; a $C_{1-6}$alkylthio-$C_{1-6}$alkyl group; a $C_{2-6}$alkenyl group; a halo-$C_{2-6}$alkenyl group; a $C_{3-6}$cycloalkenyl group; a $C_{2-6}$alkynyl group; a $C_{1-6}$alkylcarbonyl group; a $C_{1-6}$alkoxycarbonyl group; an unsubstituted phenyl group; a substituted phenyl group having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylcarbonyl groups, $C_{1-6}$alkoxycarbonyl groups, di-$C_{1-6}$alkylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; an unsubstituted phenoxy group; a substituted phenoxy group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio group; a substituted phenylthio group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkyl group; a substituted phenyl-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{2-6}$alkenyl group; a substituted phenyl-$C_{2-6}$alkenyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio

groups, halo-$C_{1-6}$alkylthio groups, $C_{1-6}$alkoxyimino-$C_{1-6}$alkyl groups and $C_{1-3}$alkylenedioxy groups; an unsubstituted phenylcarbonyl group; a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxycarbonyl group; a substituted phenoxycarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxy-$C_{1-6}$alkyl group; a substituted phenoxy-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio-$C_{1-6}$alkyl group; a substituted phenylthio-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylthio group; a substituted phenyl-$C_{1-6}$alkylthio group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylcarbonyl group; a substituted phenyl-$C_{1-6}$alkylcarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkoxycarbonyl group; a substituted phenyl-$C_{1-6}$alkoxycarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group, and

in the case of $R^4$ being a $C_{1-6}$alkyl group, $R^5$ is an unsubstituted phenylcarbonyl group or a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene groups),

in the case of X being a sulfur atom, $R^3$ is a group represented by the formula:

$-C(X^1-R^6) = N-R^7$

(wherein $X^1$ is an oxygen atom or a sulfur atom, $R^6$ is a $C_{1-6}$alkyl group, a halo-$C_{1-6}$alkyl group, a $C_{2-6}$alkenyl group, a $C_{2-6}$alkynyl group or a $C_{1-6}$alkoxy-$C_{1-6}$alkyl group, and $R^7$ is an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene

groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, and substituted phenyl groups having 1 to 5 substituents which may be the same or different and are selected from the group consisting of halogen atoms and $C_{1-6}$alkyl groups),

in the case of X being a group represented by the formula:

$N(R^8)$

(wherein $R^8$ is as defined above), $R^3$ is a group represented by the formula:

$-N=C(R^9)R^{10}$

(wherein $R^9$ and $R^{10}$, which may be the same or different, are hydrogen atoms; cyano groups; $C_{1-6}$alkyl groups; halo-$C_{1-6}$alkyl groups; $C_{3-6}$cycloalkyl groups; halo-$C_{3-6}$cycloalkyl groups; $C_{3-6}$cycloalkyl-$C_{1-6}$alkyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkoxy-$C_{1-6}$alkyl groups; $C_{1-6}$alkylthio-$C_{1-6}$alkyl groups; $C_{2-6}$alkenyl groups; halo-$C_{2-6}$alkenyl groups; $C_{2-6}$cycloalkenyl groups; $C_{2-6}$alkynyl groups; halo-$C_{2-6}$alkynyl groups; $C_{1-6}$alkylcarbonyl groups; $C_{1-6}$alkoxycarbonyl groups; unsubstituted phenyl groups; substituted phenyl groups having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{2-6}$alkenyloxy groups, halo-$C_{2-6}$alkenyloxy groups, $C_{2-6}$alkynyloxy groups, halo-$C_{2-6}$alkynyloxy groups, $C_{1-6}$alkylcarbonyl groups, $C_{1-6}$alkoxycarbonyl groups, di-$C_{1-6}$alkylamino groups, di-$C_{2-6}$alkenylamino groups, di-$C_{2-6}$alkynylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; unsubstituted phenoxy groups; substituted phenoxy groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio groups; substituted phenylthio groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkyl groups; substituted phenyl-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{2-6}$alkenyl groups; substituted phenyl-$C_{2-6}$alkenyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylcarbonyl groups; substituted phenylcarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups;

unsubstituted phenoxycarbonyl groups; substituted phenoxycarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxy-$C_{1-6}$alkyl groups; substituted phenoxy-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio-$C_{1-6}$alkyl groups; substituted phenylthio-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylthio groups; substituted phenyl-$C_{1-6}$alkylthio groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylcarbonyl groups; substituted phenyl-$C_{1-6}$alkylcarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkoxycarbonyl groups; substituted phenyl-$C_{1-6}$alkoxycarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; 5- to 7-membered heterocyclic rings having 1 to 3 heteroatoms which may be the same or different and are selected from the group consisting of oxygen atom, sulfur atom and nitrogen atom; heterocyclic rings having a benzene ring condensed therewith; or heterocyclic rings having a $C_{3-6}$cycloalkane group condensed therewith; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group), and Y is an oxygen atom or a sulfur atom.

**2.** An N-substituted phenylcarbamic acid derivative according to claim 1, wherein $R^1$ is a $C_{1-6}$alkyl group, $R^2$ is a $C_{1-6}$alkyl group, X is an oxygen atom, a sulfur atom or a group represented by the formula:

$N(R^8)$

(wherein $R^8$ is a $C_{1-6}$alkylcarbonyl group), $R^3$ is as follows:

in the case of X being an oxygen atom, $R^3$ is a group represented by the formula:

$-N=C(R^4)R^5$

(wherein $R^4$ and $R^5$ are as follows:

in the case of $R^4$ being a $C_{1-6}$alkylthio group, $R^5$ is an unsubstituted phenyl group; a substituted phenyl group having 1 to 5 substituents which may be the same or different and are selected from the group consisting of halogen atoms, $C_{1-6}$alkyl groups and $C_{1-6}$alkoxy groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, $C_{1-6}$alkyl groups and $C_{1-6}$alkoxy groups, and

in the case of $R^4$ being a $C_{1-6}$alkyl group, $R^5$ is an unsubstituted phenylcarbonyl group or a substituted phenylcarbonyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups, $C_{1-3}$alkylenedioxy groups and $C_{2-5}$alkylene groups),

in the case of X being a sulfur atom, $R^3$ is a group represented by the formula:

$-C(X^1-R^6) = N-R^7$

(wherein $X^1$ is an oxygen atom or a sulfur atom, $R^6$ is a $C_{1-6}$alkyl group, and $R^7$ is an unsubstituted phenyl group or a substituted phenyl group having 1 to 5 substituents which may be the same or different and are selected from the group consisting of halogen atoms, $C_{1-6}$alkyl groups, halo-$C_{1-6}$alkyl groups, $C_{1-6}$alkoxy groups and $C_{2-6}$alkylene groups),

in the case of X being a group represented by the formula:

$N(R^8)$

(wherein $R^8$ is as defined above), $R^3$ is a group represented by the formula:

$-N = C(R^9)R^{10}$

(wherein $R^9$ and $R^{10}$, which may be the same or different, are $C_{1-6}$alkyl groups; unsubstituted phenyl group; substituted phenyl groups having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{2-6}$alkenyloxy groups, $C_{2-6}$alkynyloxy groups, halo-$C_{2-6}$alkynyloxy groups, $C_{1-6}$alkylcarbonyl groups, $C_{1-6}$alkoxycarbonyl groups, di-$C_{1-6}$alkylamino groups, di-$C_{2-6}$alkenylamino groups, di-$C_{2-6}$alkynylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; unsubstituted phenoxy groups; substituted phenoxy groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio groups; substituted phenylthio groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkyl groups; substituted phenyl-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{2-6}$alkenyl groups; substituted phenyl-$C_{2-6}$alkenyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylcarbonyl groups; substituted phenylcarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxycarbonyl groups; substituted phenoxycarbonyl groups having 1 to 5 substituents which may be the same or different and are selected from the group consisting of halogen atoms,

cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxy-$C_{1-6}$alkyl groups; substituted phenoxy-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio-$C_{1-6}$alkyl groups; substituted phenylthio-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylthio groups; substituted phenyl-$C_{1-6}$alkylthio groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylcarbonyl groups; substituted phenyl-$C_{1-6}$alkylcarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkoxycarbonyl groups; substituted phenyl-$C_{1-6}$alkoxycarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; 5- to 7-membered heterocyclic rings having 1 to 3 heteroatoms which may be the same or different and are selected from the group consisting of oxygen atom, sulfur atom and nitrogen atom; heterocyclic rings having a benzene ring condensed therewith; or heterocyclic rings having a $C_{3-6}$cycloalkane group condensed therewith; the above heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group), and Y is an oxygen atom or a sulfur atom.

**3.** An N-substituted phenylcarbamic acid derivative according to claim 2, wherein $R^1$ is a $C_{1-6}$alkyl group, $R^2$ is a $C_{1-6}$alkyl group, X is a group represented by the formula:

$N(R^8)$

(wherein $R^8$ is a $C_{1-6}$alkylcarbonyl group), $R^3$ is a group represented by the formula:

$-N=C(R^9)R^{10}$

(wherein $R^9$ and $R^{10}$, which may be the same or different, are $C_{1-6}$alkyl groups; unsubstituted phenyl groups; substituted phenyl groups having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{2-6}$alkenyloxy groups, $C_{2-6}$alkynyloxy groups, halo-$C_{2-6}$alkynyloxy groups, di-$C_{1-6}$alkylamino groups, di-$C_{2-6}$alkenylamino groups, di-$C_{2-6}$alkynylamino groups, benzyloxy group and $C_{2-5}$alkylene groups; 5- to 7-membered heterocyclic rings having 1 to 3 heteroatoms which may be the same or different and are selected from the group consisting of oxygen atom, sulfur atom and nitrogen atom; heterocyclic rings having a benzene ring condensed therewith; or heterocyclic rings having a $C_{3-6}$cycloalkane group condensed therewith; the above heterocyclic rings being able to have one or more substituents which may be the same or different and are selected from the group consisting of halogen atoms, cyano group, nitro group, $C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups,

unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group), and Y is an oxygen atom.

**4.** An N-substituted phenylcarbamic acid derivative according to claim 3, wherein $R^1$ is a $C_{1-6}$alkyl group, $R^2$ is a $C_{1-6}$alkyl group, X is a group represented by the formula:

$N(R^8)$

(wherein $R^8$ is a $C_{1-6}$alkylcarbonyl group), $R^3$ is a group represented by the formula:

$-N=C(R^9)R^{10}$

(wherein $R^9$ and $R^{10}$, which may be the same or different, are $C_{1-6}$alkyl groups; unsubstituted phenyl groups; substituted phenyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups and $C_{2-5}$alkylene groups; or 5- to 7-membered heterocyclic rings having 1 to 3 heteroatoms which may be the same or different and are selected from the group consisting of oxygen atom, sulfur atom and nitrogen atom, said heterocyclic rings being able to have one or more substituents which may be the same or different and are selected from halogen atoms), and Y is an oxygen atom.

**5.** A process for producing an N-substituted phenylcarbamic acid derivative represented by the general formula (I):

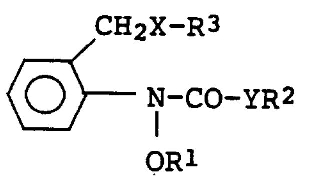

(I)

[wherein $R^1$ is a hydrogen atom, a $C_{1-6}$alkyl group, a halo $C_{1-6}$alkyl group, a $C_{1-6}$alkoxy-$C_{1-6}$alkyl group, a $C_{2-6}$alkenyl group, a $C_{2-6}$alkynyl group or a halo-$C_{1-6}$alkoxy-$C_{1-6}$alkyl group, $R^2$ is a hydrogen atom, a $C_{1-6}$alkyl group, a halo-$C_{1-6}$alkyl group, a $C_{1-6}$alkoxy-$C_{1-6}$alkyl group, a $C_{2-6}$alkenyl group, a $C_{2-6}$alkynyl group, a halo-$C_{1-6}$alkoxy-$C_{1-6}$alkyl group or a cyano-$C_{1-6}$alkyl group, X is an oxygen atom, a sulfur atom or a group represented by the formula:

$N(R^8)$

(wherein $R^8$ is a formyl group, a $C_{1-6}$alkyl group, a halo-$C_{1-6}$alkyl group, a $C_{1-6}$alkylcarbonyl group, a halo-$C_{1-6}$alkylcarbonyl group, a $C_{1-6}$alkoxycarbonyl group, a $C_{1-6}$alkoxy-$C_{1-6}$alkylcarbonyl group or a $C_{1-6}$alkylsulfonyl group), $R^3$ is as follows:

in the case of X being an oxygen atom, $R^3$ is a group represented by the formula:

$-N=C(R^4)R^5$

(wherein $R^4$ and $R^5$ are as follows:

in the case of $R^4$ being a $C_{1-6}$alkylthio group, $R^5$ is a hydrogen atom; a cyano group; a $C_{1-6}$alkyl group; a halo-$C_{1-6}$alkyl group; a $C_{3-6}$cycloalkyl group; a halo-$C_{3-6}$cycloalkyl group; a $C_{3-6}$cycloalkyl-$C_{1-6}$alkyl group; a $C_{1-6}$alkoxy group; a halo-$C_{1-6}$alkoxy group; a $C_{1-6}$alkylthio group; a halo-$C_{1-6}$alkylthio group; a $C_{1-6}$alkoxy-$C_{1-6}$alkyl group; a $C_{1-6}$alkylthio-$C_{1-6}$alkyl group; a $C_{2-6}$alkenyl group; a halo-$C_{2-6}$alkenyl group; a $C_{2-6}$cycloalkenyl group; a $C_{2-6}$alkynyl group; a $C_{1-6}$alkylcarbonyl group; a $C_{1-6}$alkoxycarbonyl group; an unsubstituted phenyl group; a substituted phenyl group having 1 to 5 substituents which may be the same or different and are selected from the group consisting of

halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkylcarbonyl groups, $C_{1-6}$alkoxycarbonyl groups, di-$C_{1-6}$alkylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; an unsubstituted phenoxy group; a substituted phenoxy group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio group; a substituted phenylthio group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkyl group; a substituted phenyl-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{2-6}$alkenyl group; a substituted phenyl-$C_{2-6}$alkenyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylcarbonyl group; a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxycarbonyl group; a substituted phenoxycarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenoxy-$C_{1-6}$alkyl group; a substituted phenoxy-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenylthio-$C_{1-6}$alkyl group; a substituted phenylthio-$C_{1-6}$alkyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylthio group; a substituted phenyl-$C_{1-6}$alkylthio group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkylcarbonyl group; a substituted phenyl-$C_{1-6}$alkylcarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; an unsubstituted phenyl-$C_{1-6}$alkoxycarbonyl group; a substituted phenyl-$C_{1-6}$alkoxycarbonyl group having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups,

$C_{1-6}$alkylthio groups, halo-$C_{1-6}$alkylthio groups, $C_{1-6}$alkoxyimino-$C_{1-6}$alkyl groups and $C_{1-3}$alkylenedioxy groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonylgroups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group, and

in the case of $R^4$ being a $C_{1-6}$alkyl group, $R^5$ is an unsubstituted phenylcarbonyl group or a substituted phenylcarbonyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene groups),

in the case of X being a sulfur atom, $R^3$ is a group represented by the formula:

$-C(X^1-R^6)=N-R^7$

(wherein $X^1$ is an oxygen atom or a sulfur atom, $R^6$ is a $C_{1-6}$alkyl group, a halo-$C_{1-6}$alkyl group, a $C_{2-6}$alkenyl group, a $C_{2-6}$alkynyl group or a $C_{1-6}$alkoxy-$C_{1-6}$alkyl group, and $R^7$ is an unsubstituted phenyl group; a substituted phenyl group having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups, $C_{1-3}$alkylenedioxy groups and $C_{2-5}$alkylene groups; a 5- to 7-membered heterocyclic ring having 1 to 3 heteroatoms which may be the same or different and are selected from the group consisting of oxygen atom, sulfur atom and nitrogen atom; a heterocyclic ring having a benzene ring condensed therewith; or a heterocyclic ring having a $C_{3-6}$cycloalkane group condensed therewith; the heterocyclic rings being able to have one or more substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, and substituted phenyl groups having 1 to 5 substituents which may be the same or different and are selected from the group consisting of halogen atoms and $C_{1-6}$alkyl groups),

in the case of X being a group represented by the formula:

$N(R^8)$

(wherein $R^8$ is as defined above), $R^3$ is a group represented by the formula:

$-N=C(R^9)R^{10}$

(wherein $R^9$ and $R^{10}$, which may be the same or different, are hydrogen atoms; cyano groups; $C_{1-6}$alkyl groups; halo-$C_{1-6}$alkyl groups; $C_{3-6}$cycloalkyl groups; halo-$C_{3-6}$cycloalkyl groups; $C_{3-6}$cycloalkyl-$C_{1-6}$alkyl groups; $C_{1-6}$alkoxy groups; halo-$C_{1-6}$alkoxy groups; $C_{1-6}$alkylthio groups; halo-$C_{1-6}$alkylthio groups; $C_{1-6}$alkoxy-$C_{1-6}$alkyl groups; $C_{1-6}$alkylthio-$C_{1-6}$alkyl groups; $C_{2-6}$alkenyl groups; halo-$C_{2-6}$alkenyl groups; $C_{2-6}$cycloalkenyl groups; $C_{2-6}$alkynyl groups; halo-$C_{2-6}$alkynyl groups; $C_{1-6}$alkylcarbonyl groups; $C_{1-6}$alkoxycarbonyl groups; unsubstituted phenyl groups; substituted phenyl groups having 1 to 5 substituents which may be the same or different and are selected from the group consisting of halogen atoms, cyano group, nitro group, formyl group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{2-6}$alkenyloxy groups, halo-$C_{2-6}$alkenyloxy groups, $C_{2-6}$alkynyloxy groups, halo-$C_{2-6}$alkynyloxy groups, $C_{1-6}$alkylcarbonyl groups, $C_{1-6}$alkoxycarbonyl groups, di-$C_{1-6}$alkylamino groups, di-$C_{2-6}$alkenylamino groups, di-$C_{2-6}$alkynylamino groups, unsubstituted phenoxy group, substituted phenoxy groups having 1 to 5 substituents which may be the same or different and are selected from the group consisting of halogen atoms, $C_{1-6}$alkyl groups, $C_{1-6}$alkoxy groups and cyano

group, unsubstituted heteroaryloxy groups, substituted heteroaryloxy groups having 1 to 5 substituents which may be the same or different and are selected from the group consisting of halogen atoms, $C_{1-6}$alkyl groups, $C_{1-6}$alkoxy groups and cyano group, unsubstituted benzyloxy group, substituted benzyloxy groups having 1 to 5 substituents which may be the same or different and are selected from the group consisting of halogen atoms, $C_{1-6}$alkyl groups, $C_{1-6}$alkoxy groups and cyano group, $C_{1-6}$alkoxyimino-$C_{1-6}$alkyl groups, $C_{1-3}$alkylenedioxy groups, and $C_{2-5}$alkylene groups; unsubstituted phenoxy groups; substituted phenoxy groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio groups; substituted phenylthio groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkyl groups; substituted phenyl-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{2-6}$alkenyl groups; substituted phenyl-$C_{2-6}$alkenyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylcarbonyl groups; substituted phenylcarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxycarbonyl groups; substituted phenoxycarbonyl groups having 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenoxy-$C_{1-6}$alkyl groups; substituted phenoxy-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenylthio-$C_{1-6}$alkyl groups; substituted phenylthio-$C_{1-6}$alkyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylthio groups; substituted phenyl-$C_{1-6}$alkylthio groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkylcarbonyl groups; substituted phenyl-$C_{1-6}$alkylcarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; unsubstituted phenyl-$C_{1-6}$alkoxycarbonyl groups; substituted phenyl-$C_{1-6}$alkoxycarbonyl groups having on the ring 1 to 5 substituents which may be the same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxyimino-$C_{1-6}$alkyl groups and $C_{1-3}$alkylenedioxy groups; 5- to 7-membered heterocyclic rings having 1 to 3 heteroatoms which may be the same or different and are selected from the group consisting of oxygen atom, sulfur atom and nitrogen atom; heterocyclic rings having a benzene ring condensed therewith; or heterocyclic rings having a $C_{3-6}$cycloalkane group condensed therewith; the above heterocyclic rings being able to have one or more substituents which may be the

same or different and are selected from the group consisting of halogen atoms, cyano group, nitro group, $C_{1-6}$alkyl groups, halo-$C_{1-6}$alkyl groups, $C_{1-6}$alkoxy groups, halo-$C_{1-6}$alkoxy groups, $C_{1-6}$alkylthio groups, halo-$C_{1-6}$alkylthio groups, $C_{1-6}$alkoxycarbonyl groups, unsubstituted phenyl group, substituted phenyl groups having 1 to 5 substituents which may be the same or different and are selected from the group consisting of halogen atoms and $C_{1-6}$alkyl groups, phenyl-$C_{1-6}$alkyl groups, pyridyl group, pyrimidyl group and dioxolane group), and Y is an oxygen atom or a sulfur atom] which comprises reacting a compound represented by the general formula (III):

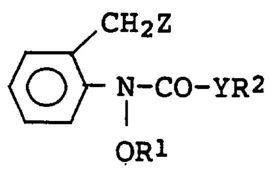

(III)

(wherein $R^1$, $R^2$ and Y are as defined above, and Z is a halogen atom) with a compound represented by the general formula (II):

$H\text{-}X\text{-}R^3$ (II)

(wherein X and $R^3$ are as defined above).

**6.** An agricultural and horticultural fungicide comprising an N-substituted phenylcarbamic acid derivative set forth in claim 1 as an active ingredient.

**7.** An agricultural and horticultural fungicide comprising an N-substituted phenylcarbamic acid derivative set forth in claim 2 as an active ingredient.

**8.** An agricultural and horticultural fungicide comprising an N-substituted phenylcarbamic acid derivative set forth in claim 3 as an active ingredient.

**9.** An agricultural and horticultural fungicide comprising an N-substituted phenylcarbamic acid derivative set forth in claim 4 as an active ingredient.